(19) Europäisches Patentamt
European Patent Office
Office européen des brevets

(11) **EP 2 323 603 B1**

(12) **EUROPEAN PATENT SPECIFICATION**

(45) Date of publication and mention
of the grant of the patent:
**04.12.2019 Bulletin 2019/49**

(21) Application number: **09810536.4**

(22) Date of filing: **26.08.2009**

(51) Int Cl.:
**A61F 13/08** (2006.01)

(86) International application number:
**PCT/US2009/055051**

(87) International publication number:
**WO 2010/025186 (04.03.2010 Gazette 2010/09)**

(54) **THERAPEUTIC COMPRESSION GARMENTS**

THERAPEUTISCHE KOMPRESSIONSKLEIDUNG

VÊTEMENTS DE COMPRESSION THÉRAPEUTIQUES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **28.08.2008 US 92459 P**
**23.02.2009 US 391051**

(43) Date of publication of application:
**25.05.2011 Bulletin 2011/21**

(73) Proprietor: **Farrow Innovations LLC**
**Bryan TX 77803 (US)**

(72) Inventors:
• **FARROW, Wade, P.**
**College Station**
**Texas 77845 (US)**

• **CREIGHTON, Barry, L.**
**College Station**
**Texas 77845 (US)**

(74) Representative: **Tomkinson, Alexandra**
**Bailey Walsh & Co LLP**
**1 York Place**
**Leeds, LS1 2DR (GB)**

(56) References cited:
**EP-A1- 1 459 717**     **WO-A1-93/14730**
**US-A- 3 856 008**     **US-A- 4 562 834**
**US-A- 4 829 993**     **US-A1- 2003 088 201**
**US-A1- 2006 010 574**     **US-A1- 2007 179 421**
**US-A1- 2007 276 310**

**Description**

TECHNICAL FIELD

This disclosure relates to apparatus for treating medical conditions by application of controlled compression to general and specific areas of a patient's body.

BACKGROUND OF THE INVENTION

Excessive interstitial fluid accumulation, referred to as edema, may arise from a variety of illnesses and conditions, including venous valvular insufficiency, postphlebotic syndrome, and lymphedema. Compression apparatus method and systems control edema by reduction of interstitial fluids which increases nutrient delivery to tissues, removes waste from tissues, relieves pain from swelling, and decreases risk of infection. However, prior-art compression technologies have certain drawbacks as explained below.

Wounds complicate the problem because traditional compression apparatus may restrict drainage of fluid from sores, cause skin breaks and/or ulcerations, and may promote wound breakdown and increased risk of blood clot formation in the veins.

Clinically, certain patient populations develop pressure necrosis to underlying skin and tissue breakdown occasionally occurs with traditional modalities, including compression stockings. This occurs most commonly over the anterior ankle where the tibialis tendon can be very prominent in some individuals. Some patients have a tibia which is prominent and plough-shaped, such that these patients can experience breakdown across parts of the shin area under these garments. Similarly, the malleoli and metatarsal heads of the 1st and 5th digits are occasional problems as well.

Some patients have troublesome anatomical morphologies, such as large bunions, metatarsal head protrusions, or accentuated ankle malleoli, which are predisposed to higher peak compression levels.

Some patients requiring compression have fragile skin that cannot tolerate even moderately elevated compressive or shear forces.

Patients with lymphedema and venous insufficiency often develop fibrotic areas within the tissues or may be lacking in lymphatic integrity

Many of the above mentioned patients are prone to bacterial and fungal skin infections, all of which can be life threatening.

Particularly problematic in venous and lymphedema patients and other patients requiring long term compression is the incidence of dermatitis, causing itching, and discomfort which contribute to lack of compliance with compression and is therefore also detrimental to attempts at edema reduction and healing of wounds in edematous limbs.

Areas of high flexion such as the elbow, knee and anterior ankle may require greater bending of the apparatus and better breathability than is traditionally provided. Also, sensitive arteries, veins, and nerves that course along the posterior of the leg when the knee is bent require better protection from impinging garment structures during flexion than what is conventionally provided.

Because of considerable variation in limb shapes and sizes, custom garments may traditionally be required. However, conventional custom garments take time to manufacture. It is not uncommon, for instance, for conventional custom compression stockings to take one month from date of order until the patient receives the garment. Furthermore, errors in manufacturing and measurement sometimes necessitate remanufacturing the garment or altering the garment in order to get a proper fit. This is very inconvenient for the patient, who needs therapeutic compression immediately, and must make-do with an off-the-shelf garment or bandage until the custom garment arrives and fits correctly.

RELATED ART

Many devices can be used for treatment of edema, swelling, or venous ulcerations. For example, the Unna boot, invented by German dermatologist Dr. Unna in the late 1800's for use in the treatment of venous ulcerations, uses a zinc paste bandage which dries to form an inelastic shell around the limb. When a calf muscle expands on activation the muscle cannot expand against this rigid shell. Thus high subbandage pressure redirects the pressure inward where it exerts force on the deep venous system, augmenting venous return. While useful in some applications, the Unna boot is subject to a number of limitations. For example, it is applied as a rigid shell. Therefore, controlled baseline compression is difficult to ascertain because there is no feedback to guide when appropriate compression levels are obtained. Furthermore, as edema reduces, the boot loses compression and allows bandages to shift, possibly increasing drainage in the case of any wounds present.

A further limitation is that the rigid shell boot can cause pressure ulceration if it dries with too much pressure over bony prominences or other sensitive tissue areas. Patients undergoing total contact cast therapy are commonly unable to feel when a rigid cast is not properly applied causing dangerous pressure points or when padding is insufficient, exposing the skin to the rough inner surface of the cast.

Circaid and LegAssist have produced nonelastic garments for treatment of swelling, basically recreating a removable nonelastic garment with performance similar to the Unna boot. There are limitations to these type garments. The garment does not form fit well as it is relatively nonelastic. The garment loosens as edema reduces, and requires more frequent readjustments, and inelastic products do not function well over joints Finally, there is no intuitive way that a user can control the baseline or resting compression as there is no feedback from the garment.

Some products use layers of long and mid-stretch bandages in combination to create a flexible shell which has some elasticity, provides padding to the skin, and can apply fairly uniform compression. A four layer compression system invented in the 1980s by Dr. Christine Moffatt is a single use disposable bandage system. Multilayer compression systems are now available in 2 to 4 layers by many companies. However, properly trained and experienced health care practitioners are required for application of these systems to insure controlled compression and to effect safe distal to proximal compression gradients.

The Coban 2 layer bandage system is such a system with a single use disposable bandage. This garment shows promise in treatment of oedemas, due to lower profile and studies showing decreased slippage over a one week period. The garment can be applied at less than maximal stretch (ex. Applied at 50% or 75% of possible maximal stretch - i.e. 50% of maximal stretch in this case means bandage applied at 15% stretch) to reduce compression level. The system features an inner layer with a thin layer of foam with a layer of cohesive bandage and very little compression, and an outer layer with approximately 30% maximum stretch (bandage stretches 130% original length) which is designed to provide therapeutic compression. The system is lower profile than multilayer bandages, and many users feel it allows them to have easier ambulation and lower profile under pants as well. The foam under layer is considered padding and helps reduce slippage, but is also applied over the entire limb. The Coban 2 layer compression bandage system, however, sometimes does not provide adequate padding to certain problems areas, and extra padding must be used or tissue breakdown can result.

Padding can also be useful to help reduce swelling in problem areas. This is seen for example in the retromalleolar area, which can be difficult to contain with compression garments or bandages without additional padding to help press in and increase interstitial pressures, effectively augmenting return of fluid to the capillaries and lymphatics. Therefore, padding under compression garments may prevent trauma to underlying tissues.

Although systems on the market exist which provide chipped foam either in channels or squares, none mix smooth foam for bony circumferences with the channeled and/or chipped foam liners.

For lymphedema patients, solid and chipped foam liners for padding are known in the art. These are sold under the name Circaid Silhouette, Jovi, Solaris and others.

Higher quality liners use specific foam densities to provide better padding and protection of underlying tissues. Some liners may contain channeling, which is felt by many experts to help channel flow of lymph fluid by utilizing areas of higher compression and areas of lower compression.

Most current solutions provide chipped foam sewn into a sleeve with channeling. These products are thick, and some patients complain they are hot and bulky to wear on a day in and day out basis. These solutions are expensive, and are mainly used for treatment of lymphedema and fibrotic tissue caused by lipodermatosclerosis, for which many experts find these products useful. Additionally, the excess bulk means that regular pants often cannot be worn over such garments, and if worn over a joint tend to restrict the motion in that joint. Because of these limitations, such garments are typically worn at night and most patients do not use them for everyday use. Other solutions include smooth foam liners, however, these also suffer from being very insulative and bulky, not to mention expensive because the foam is applied throughout the liner.

Conventional compression bandages that employ short-stretch material or that have short-stretch properties are advantageous because they allow different therapeutic compression ranges at end-stretch and allow prescribers to properly dictate the level of resting compression best suited for the patient. However, these bandages must be applied by a practitioner who has been properly trained and practiced in the amount of tension that should be applied. Otherwise the patient may be harmed by resulting circulation problems if the bandages are applied with too much tension.

In light of the foregoing, there is a need in the art for compression apparatus, methods and systems that solve these and other issues in the prior art.

## SUMMARY OF THE INVENTION

In accordance with aspects of the present invention, an apparatus, method and system for facilitating therapeutic compression having controlled and repeatable baseline compressions that provide intuitive feedback to patients so that they may safely apply proper compression, and proper distal to proximal compression gradients to themselves without the need for frequent visits to clinics for trained practitioner services, is presented.

In accordance with further aspects of the present invention, an apparatus, method and system for facilitating therapeutic compression that form-fit well and do not loosen as edema reduces and do not require frequent readjustments, is presented.

In accordance with still further aspects of the present invention, an apparatus, method and system for facilitating therapeutic compression that conform to patients having different anatomical morphologies, such as large bunions, metatarsal head protrusions, or accentuated ankle malleoli, which are predisposed to higher peak compression levels, without developing pressure necrosis to underlying skin or causing tissue breakdown, is presented.

In accordance with yet further aspects of the present invention, an apparatus, method and system for facilitating therapeutic compression by adapting over the anterior ankle where the tibialis tendon can be very prominent, and tibia which are prominent and plough-shaped in some individuals, and the malleoli and metatarsal heads of the 1st and 5th digits, without experiencing breakdown or necrosis in these areas, is presented.

In accordance with still further aspects of the present invention, an apparatus, method and system for facilitating therapeutic compression to patients having fragile skin who cannot tolerate even moderately elevated compressive or shear forces, and patients with lymphedema or venous insufficiency, or patients who may be lacking in lymphatic integrity, and patients that are prone to fungal, bacterial, and viral skin infections, without causing fibrotic areas within the tissues and without introducing other deleterious conditions that are often associated with conventional systems, is presented.

In accordance with still further aspects of the present invention, an apparatus, method and system for facilitating therapeutic compression to lymphedema patients and patients requiring long term compression without causing incidences of dermatitis, itching, and discomfort which contribute to lack of compliance with compression in patients and is therefore also detrimental to attempts at edema reduction and healing of wounds in edematous limbs, is presented.

In accordance with yet further aspects of the present invention, an apparatus, method and system for facilitating therapeutic compression to patients with wounds without restricting drainage of fluid from sores which may cause skin breaks, and/or ulcerations and without which may promote wound breakdown and risk of blood clot formation in the veins, is presented.

In accordance with still further aspects of the present invention, an apparatus, method and system for facilitating therapeutic compression to areas of high flexion such as the elbow, knee and anterior ankle, by providing greater bending, better breathability and better protection from impinging garment structures during flexion, while protecting sensitive arteries, veins, and nerves that course along the posterior of the leg, is provided.

In accordance with still further aspects of the present invention, an apparatus, method and system for facilitating therapeutic compression to patients who require custom garments or custom compression stockings with garment systems that are customizable at the point of sale by a durable medical equipment company, clinic, hospital on site, or even by the patients themselves so that the garment is immediately available with a correct fit, is provided.

In yet a further aspect of the present invention, therapeutic electrical stimulation of muscles, particularly for patients with limb paralysis or other reasons resulting in lack of mobility, for actuating muscle contraction in an affected limb while the limb is in a short-stretch compression garment, such as FarrowWrap, thereby activating natural muscle pump functions to reduce edema, is presented.

The invention is an apparatus as disclosed in claims 1-8 herein.

The invention is shown in specific embodiments with reference to figures 38 to 41 and 53 to 56.

BRIEF DESCRIPTION OF THE DRAWINGS

Fig. 1 is an illustration of one exemplary arrangement of padding over an anterior tibial crest.

Fig. 2 is an illustration of padding over anterior tibial crest and anterior ankle.

Fig. 3 is an illustration of padding over anterior tibial crest, anterior ankle, and malleoli.

Fig. 4 is an illustration of padding over anterior tibial crest, anterior ankle, malleoli, and metatarsals.

Fig. 5 is an illustration of a detailed view of padding over anterior tibial crest.

Fig. 6 is an illustration of a detailed view of padding over anterior tibial crest.

Fig. 7 is an illustration of a detailed view of padding over anterior tibial crest.

Fig. 8 is an illustration of a detailed view of padding over anterior tibial crest.

Fig. 9 is an illustration of a detailed view of padding over malleolus.

Fig. 10 is an illustration of a detailed view of padding over malleolus.

Fig. 11 is an is an illustration of a detailed view of padding over malleolus.

Fig. 12 detailed view of padding over the anterior ankle.

Fig. 13 is an illustration of padding over anterior ankle and dorsal foot.

Fig. 14 is an illustration of padding on the metatarsal area.

Fig. 15 is an illustration of padding over anterior ankle and dorsal foot.

Fig. 16 is an illustration of padding over malleoli.

Fig. 17 is an illustration of padding over anterior ankle, and malleoli

Fig. 18 is an illustration of padding over anterior ankle, dorsal foot.

Fig. 19 is an illustration of the anterior ankle, dorsal foot, malleoli, and metatarsal areas.

Fig. 20 is an illustration of a garment with channeled padding on arm.

Fig. 21 is an illustration of padding over anterior ankle, dorsal foot, and malleoli.

Fig. 22 is an illustration of padding over anterior tibial crest, anterior ankle, dorsal foot, malleoli, and channeled padding knee high.

Fig. 23 is an illustration of padding over anterior tibial crest, anterior ankle, dorsal foot, malleoli, and channeled padding knee high.

Fig. 24 is an illustration is an illustration of padding over anterior tibial crest, anterior ankle, dorsal foot, malleoli, and channeled padding knee high.

Fig. 25 is an illustration of a garment with channeled padding on thigh.

Fig. 26 is an illustration of a garment with channeled padding on thigh.

Fig. 27 is an illustration of a garment with channeled padding on thigh.

Fig. 28 an illustration of padding over anterior tibial crest, anterior ankle, dorsal foot, malleoli, and channeled padding knee high.

Fig. 29 is an illustration of padding over anterior tibial crest, anterior ankle and dorsal foot with channeled padding thigh high.

Fig. 30 is an illustration of channeled padding.

Fig. 31 is an illustration of channeled padding.

Fig. 32 is an illustration of waffled padding.

Fig. 33 is an illustration of waffled padding.

Fig. 34 is an illustration of continuous rolls of padding.

Fig. 35 is an illustration of continuous rolls of padding.

Fig. 36 is an illustration of a system comprising a liner and a compression garment.

Fig.37 is an illustration of another padded liner embodiment containing padding over entire calf and over the dorsal foot area.

Fig. 38 is an illustration of an embodiment of a trim-to-fit footpiece.

Fig. 39 is an illustration of an embodiment of a trim-to-fit legpiece.

Fig. 40 is an illustration of an embodiment of a trim-to-fit legpiece.

Fig. 41 is an illustration of an embodiment of a trim-to-fit legpiece.

Fig. 42 is an illustration of a kneepiece.

Fig. 43 is an illustration of a kneepiece.

Fig. 44 is an illustration of a middle band of the kneepiece of Fig. 43.

Fig. 45 is an illustration of a spine of the kneepiece of Fig. 43.

Fig. 46 is an illustration of a middle band and straight bands of a kneepiece.

Fig. 47 is an illustration of a backside view of the kneepiece of Fig. 43.

Fig. 48 is an illustration of a kneepiece with detachable components.

Fig. 49 is an illustration of a kneepiece.

Fig. 50 is an illustration of a kneepiece.

Fig. 51 is an illustration of a kneepiece.

Fig. 52 is an illustration of a kneepiece on a patient's leg.

Figs. 53a-d are illustrations of embodiments of a Choice Algorithm.

Fig. 54 is an illustration of a Choice Algorithm table.

Fig. 55 is an illustration of a form usable in a Trim-to-Fit business method.

Fig. 56 is an illustration of a liner about a limb with cross-sections of the liner taken at different levels.

Fig. 57a-c are illustrations of a band having a trapezoidal shape and the band being applied about a limb.

Fig. 58 is an illustration of a garment using some trapezoidal shape bands

## DETAILED DESCRIPTION

The present disclosure relates generally to treatment of edema and, more specifically, to a liner to be used under a device for applying compressive pressure to a person's body in order to facilitate reduction of interstitial fluids from a body trunk and/or limb extremity and to provide support and fatigue relief.

It is to be understood that the present disclosure provides many different embodiments, or examples, for implementing different features of various embodiments. Specific examples of components and arrangements are described below to simplify the present disclosure. These are, of course, merely examples and are not intended to be limiting. In addition, the present disclosure may repeat reference numerals and/or letters in the various examples. This repetition is for the purpose of simplicity and clarity and does not, in itself, dictate a relationship between the various embodiments and/or configurations discussed. Moreover, the formation of a first feature over or on a second feature in the description that follows may include embodiments in which the first and second features are formed in direct contact, and may also include embodiments in which additional features may be formed interposing the first and second features, such that the first and second features may not be in direct contact.

This disclosure details a liner with padding over prominent bony areas. This liner is designed to be very low profile and breathable and to be worn under a compression garment such as a FarrowWrap™ short-stretch active compression garment. The liner is very important, as it allows higher resting levels of compression to be used safely by decreasing skin surface pressure to bony areas by padding these areas, while maintaining lower profile and higher breathability to areas that do not need this padding. The Sub-Bandage Pressure is the pressure exerted just under the garment. In the absence of padding, the Skin Surface Pressure is same as the subbandage pressure. The more padding there is between the compression bandage and the skin, the lower the skin surface compression. Thus, a liner reduces skin surface pressure by lowering the pressure over the bony / tendinous area low enough that capillary perfusion pressure is not compromised. Compromising capillary perfusion pressure would result in tissue areas which were not adequately perfused and lead to tissue ischemic damage and tissue loss.

The liner can be manufactured in a variety of ways. One method is to use a continuous roll of liner material as is known in the art. The padding covering the necessary areas would be attached selectively to the liner. This may be from methods such as sewing or RF or ultrasonic welding. Alternatively, the padding may be sewn in place or an industrial fabric glue appropriate for this use may be used.

The padding may be flat or with linear grooves or cross-hatching. It may be made of foam or spacer fabric or one or more layers or thick terry-cloth material.

Alternatively, the liner may be made with a circular knit machine with the padding woven in to these necessary areas, such as a padded sock, compression stocking, or liner with spacer fabric padding. A circular knit machine is often used to make spacer fabric, and the machine could produce liners with spacer fabric padding only in desired areas over bony prominences, or to provide the channeling.

Preferably this padding would be 0.1 - 2cm thick, depending on the embodiment, although other ranges are possible. In some applications the padding may need to be thicker or higher density material than for other applications. The edges may be beveled to allow a transition to the non-padded areas.

Some embodiments would necessitate more padding to some areas of the liner than other areas. For instance, a knee high liner with thin 0.2cm padding over the tibia and then a thicker 0.8cm padded area over the anterior ankle to better protect the tibialis may be necessary for some embodiments.

In some embodiments the padding would be selectively removable from the liner, either in a knitted or sewn on pouch, or attached selectively with Velcro®-like hook and loop or another sticky substance, or a variety of other methods as are known in the art.

In some embodiments the padding would be beveled at the edges of the padding.

In some embodiments, the padding would comprise of one or more layers of circular spacer fabric. This fabric has two layers of fabric with mostly air in-between and held in place by the orientation of threads between the fabric layers and is created on a circular knit machine. Such technology is emerging now, and available from Beverly Knits Inc. in Charlotte, NC and its construction is detailed in US Patent 6,755,052.

Other manufacturers of spacer fabric include 3Mesh by Mueller Textiles of Wiehl, Germany; Spacetec by Heathcoat Fabrics Ltd, Devon, UK; AirX by Tytex, Ikast, Denmark; XD Spacer Fabric by Changshu Jianhua Knitting Company, Jiangsu, China; and XD Spacer Fabric by Baltex, Derbyshire, UK.

Different types of spacer fabric may be used with a variety fibers, filaments or monofilaments between layers, and various densities of fibers, and length of fibers depending on application. For instance more compression resistance is better for fibrotic tissue. Other examples may include thicker padding under tighter compression over certain body parts like the tibialis tendon which varies in prominence. Depending on the application varying stretch of fabric layer(s) may be needed if the entire padding is made of spacer fabric.

In some embodiments, the spacer fabric would be knit on a programmable or computer programmable circular knit machine, with spacer fabric only in designated areas, and in other areas single layer or two layers fabric may be used in the liner.

The spacer fabric has many interesting applications for use as an alternative to foam. Because it is highly breathable but has compression resistance, it may be superior to foam for use as padding under compression garments, casts, and orthotics. The higher breathability means less skin moisture, which decreases incidence of dermatitis and decrease risk of fungal infection. This is particularly important in lymphedema patients and patients requiring long term compression, where the incidence of dermatitis, itching, and discomfort contributes to lack of compliance with compression in patients and is therefore also detrimental to attempts at edema reduction and healing of wounds in edematous limbs.

Alternative materials to spacer fabric include open cell foam, closed cell foam, viscoelastic foam, fluid filled elastic enclosed padding, silicon padding, terry cloth, wadded fabric, flexible aerogel blanket, pressurized gas, injected foam (single or multipart foam), down, mechanical (adjustable spring - metal, nonmetal), inorganic polymers, dilatant fluids whose viscosity changes with applied strain, synthetic and natural rubber, magnetic (like poles repel) multilayer fabrics with tiny Neodimium-Iron-Boron NIB magnets sewn or bonded to the layers such that the like magnetic poles are facing each other (one can achieve good repulsion by using high quality NIB magnets).

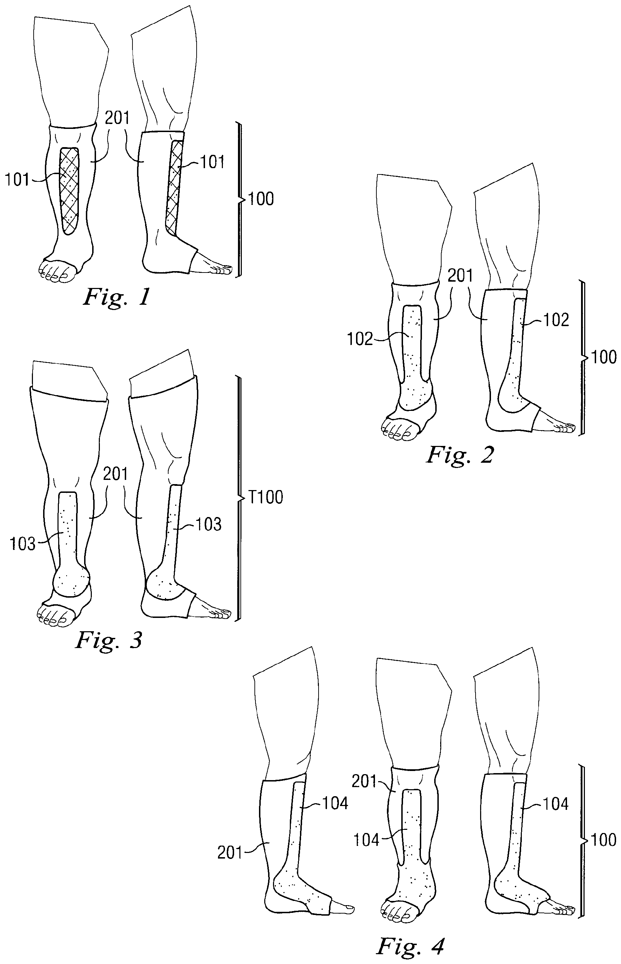

Referring to Figs. 1-4, the present disclosure shows a number of exemplary liner shapes 100 and T100 disposed about a limb, in this case a leg of a patient. These Figures each show a front view and a right side view. Fig. 4 also shows a left side view of the liner shape 100. The liners in these examples are formed as tubular sleeves that may be donned about the limb, having an opening at both ends. Other liners are stocking-like having only a single opening and closed toe. These liners each include a first region (referred to as 101, 102, 103, 104) and a second region (referred to as 201) that differ in the amount of padding provided. In these examples, the second region 201 comprises more padding than the first regions 101, 102, 103, 104. As shown in Figs. 1-4, the padded first regions 101, 102, 103, 104, overlies prominent bony areas on the patient's limb. These liners (100 and T100) have a very low profile in the second region 201, having less padding, also providing high breathability.

The liner material may be any traditional material as known in the art, from terry cloth type material, to high performance techsheen with compression, to conventional sock or compression stocking materials with flat-knit or circular or other construction with 8 - 50mmHg compression, to product similar to Comperm brand tubing with low grade compression, or a stockingette material that comes in a roll, to a sock type construction. The padding that forms the first regions 101, 102, 103, 104 may be sewn in place in strategic locations to reduce skin surface pressures and increase comfort and decrease risk of pressure necrosis to underlying skin. Alternatively, the padding may be knitted or woven into place during liner construction, or selectively detachable.

The padded area of the first region may be attached to the liner material by any of many known techniques and may be selectively detachable, permanently attached, or temporarily attached. Alternatively, the padding may be separate from the liner.

Methods of attachment may include sewing, buttons, snaps, RF or ultrasonic welding, gluing, laminating, or hook and loop type attachments, among others as are known in the art. Another method of attachment may be a pouch for the liner to slide into, or fabric glue to hold padding against liner. Alternatively, the padding may be knitted into place and contiguous with the liner material.

Fig. 1 details one embodiment of a liner shape 100 of the current disclosure. The prominence of the anterior tibial crest varies, but in some patients is sharp, with little overlying soft tissue. Attempts to place compression on the limb results in pressure necrosis and pain over the soft tissues over the anterior tibial crest. Padding to this area, as shown in the padded region 101 reduces skin surface pressure and reduces complications and increases comfort. The second region 201 details the liner material in conjunction for use with the padded area.

Fig. 2 details another embodiment of a liner shape 100 of the disclosure, where the padded areas of the first region 102 provide protection to the anterior tibial crest as well as the anterior ankle area. The liner shape 100 details a knee high embodiment of the associated liner material 201, which could also represent a thigh high, arm, or other garment as known in the art. In the inventor's experience providing therapeutic compression to thousands of patients, the anterior ankle is the most common location for pressure necrosis and pain under compression garments, compression bandages, and orthotics. This is due to the prominence in some patients of the tibialis tendon, and the sharp bend in the leg at this anatomical location.

Fig. 3 shows another embodiment of a liner shape T100 of the current disclosure, where the padded areas of the first region 103 include the anterior tibial crest, the anterior ankle, as well as the malleoli and an associated liner material forming the second region 201, here shown as a thigh high garment T100, although other embodiments, such as an arm, are possible.

Fig. 4 shows an embodiment of a liner shape 100 where the liner shape 100 provides padding in the first region 104 to all the highest risk areas in the lower limb, including the anterior tibial crest, anterior ankle, malleoli, and the first and fifth metatarsals. As with the prior embodiments, the second region 201 is less padded and may include only unpadded liner material.

Note that while the embodiment of liner shape 100 in Fig. 4 protects all the highest risk areas, it still covers very little surface area of the lower leg in which liner material 201 covers much less than 50% and more likely 33% - 20% of the surface area of the limb below the knee. Likewise, all the embodiments herein include a padded first region 104 that covers less than 50%, and more likely between 5%- 33% of the surface area of the liner on the limb. This reduces material consumption resulting in cost efficiencies, and also provides a lower profile and higher breathability to the wearer, which translates into increased patient safety and comfort.

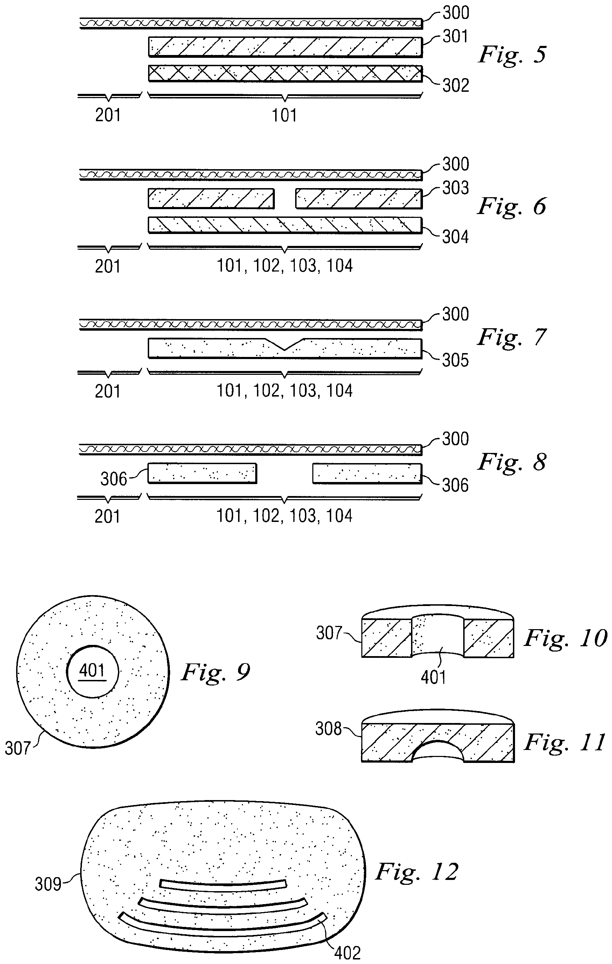

Fig. 5 shows a detail drawing cross-section of one embodiment of the liner shape 100 illustrated in Fig. 1. This cross-section shows liner material 300 that forms the second region 201 and one or more padding layers that cooperate to form the first region 101. The first region 101 padding layers here are shown as two layers of padding material 301 and 302. In this illustration, the two padding layers may be attached to the liner material 300 by any known means such as sewing, weaving or knitting them together at time of construction, RF or ultrasonic welding, lamination. The materials 301 and 302 may be beveled at the edges to allow smooth transition to the non-padded areas of the unpadded region 201. Furthermore, the outer layer (shown here as 302) may have less width to create a stair-step type beveling of the material. It is understood that more or less layers may be necessary, depending on the padding material chosen. Here, it is contemplated that any suitable padding may be used, including for example, spacer fabric, and thickness will depend on application and compressibility of the materials.

Fig. 6 shows another exemplary cross-section of a liner shape 100, that may be used as shown in any of Figs. 1-4. In this case, there is shown 2 padding layers 303 and 304, with part of the padding missing in the layer 303. This is to reduce skin surface pressures over the anterior tibial crest. This groove may help hold the padding in the proper place, and also help distribute pressure more equally over a larger surface area by applying less padding over the middle of the anterior tibial crest and more on the sides, reduces the total pressure per unit area. This reduction in pressure per unit area may reduce risk of pressure necrosis and increase comfort. This may also improve breathability of the padding further.

Fig. 7 shows another embodiment of a liner shape100 that may be used as shown in any of Figs. 1-4. Here, the padding 305 forming first region 101,102,103,104 includes a wedge-shaped groove cut from the inside of the padding. The liner material 300 forms second region 201 and may or may not follow the wedge. In this drawing, it does not come in contact with the groove.

Fig. 8 shows another embodiment of a liner shape100 that may be used as shown in any of Figs. 1-4, where two strips of padding 306, forming regions 101, 102, 103, 104, are used instead of one large one as in Fig. 7. Again, this embodiment can help reduce pressure to the anterior tibial crest area and redistribute to either side.

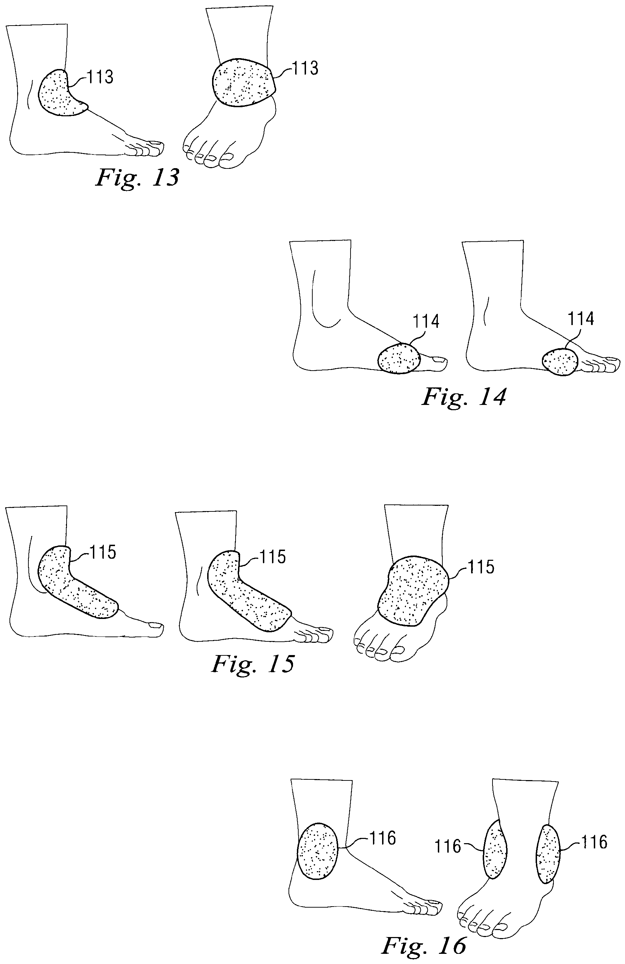

Fig. 9 shows one exemplary embodiment of padding 307 that forms the padded first region in any of the liners discussed herein with application to the malleoli (see Fig. 13) or metatarsal areas (Fig. 11). In this illustration, there is a hole 401 in the padding 307. As shown in Fig. 10, this hole may go all the way through the middle of the padding 307, or shown as a cross-section 308 in Fig. 11 may be beveled. The padding may have beveling on the outer edges, which is not illustrated in this drawing. Again, the reduced padding in the middle helps redistribute pressure more equally to a larger surface area and reduce pressure on the malleoli or metatarsals.

Fig. 12 shows an exemplary embodiment of the padding 309 usable to form the padded first region formed to accommodate curvature along a body part. In this case, the padding 309 accommodates the anterior ankle (see Fig. 13). In Fig. 12 one or more slits or grooves 402 in padding 309 are cut or stamped into the padding.

This groove or grooves 402 may extend only partway through the material, or may extend all the way through the material. These grooves 402 facilitate the bending of the padding and provide more even reduction of the skin surface pressures. The grooves 402, or slits, would preferentially follow the outline of the curvature of the ankle, as shown in this illustration. Other embodiments are possible, such as slits that go vertically and follow better the course of the tibialis tendon. In other embodiments, holes can be used instead of grooves or slits. By changing the number of holes, density of holes, and size of each hole, greater breathability, better conformity of the padding to the underlying skin curvature, and better reduction of skin surface pressure can be created. By modeling the pressure over a number of patients, one can engineer the best size and location of these grooves or holes and then use known methods of manufacture to place or create or mold the holes, slits, or grooves in the correct location of the padding in order to maximize comfort, breathability, conformability of the padded liner, and pressure reduction.

Fig. 13 and the Figures making up Figs. 14-19 each show the padded first region of the liner as located upon a limb, such as the foot. Although not discussed further with respect to these figures, each padded first region is associated with a less padded second region of a liner that covers at least a portion of the limb in the manner discussed above. These drawings however, are intended to show the location of the padded first region relative to a patient's limb, such as a foot.

Fig. 13 shows the padded first region 113 aligned such that the padding provides protection to the anterior ankle as well as the dorsal foot areas. In this case, the dorsal foot padding is often needed to facilitate edema reduction better under the overlying compression device rather than reducing skin surface pressures. The padding, such as foam, may be shaped in such a way to provide maximum therapeutic compression to the dorsal foot area.

Fig. 14 shows a padded first region 114 disposed on the metatarsal areas as discussed above with reference to Fig. 9. Fig. 15 shows a padded first region 115 disposed to provide padding to the anterior ankle and the dorsal foot. Fig. 16 shows a padded first region arranged to provide padding to the malleoli as discussed above. Fig. 16, shows that the padded first region 116 may be divided or separated into a plurality of separate padded regions.

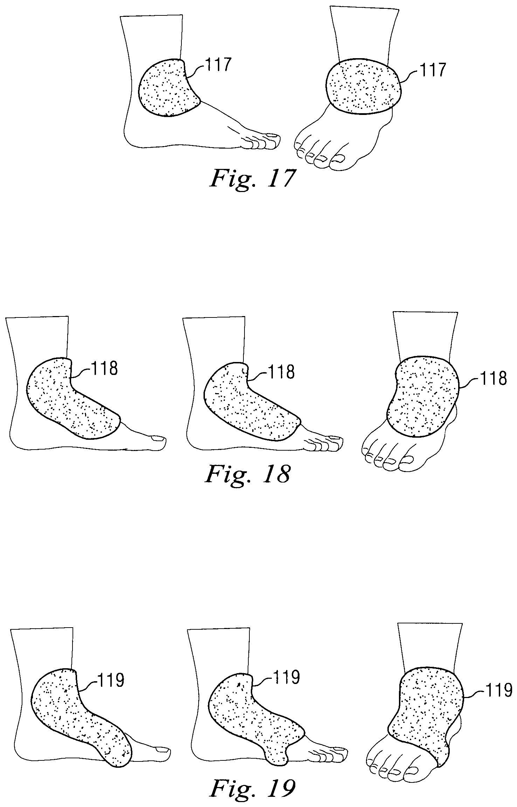

Fig.17 shows an embodiment where the padding of the first region 117 provides protection just to the anterior ankle and malleoli areas. Fig. 18 shows an embodiment where the padding of the first region 118 provides protection to the anterior ankle and dorsal foot.

Fig. 19 shows an embodiment where the padding 119 provides protection to the anterior ankle, dorsal foot, malleoli, and metatarsal areas.

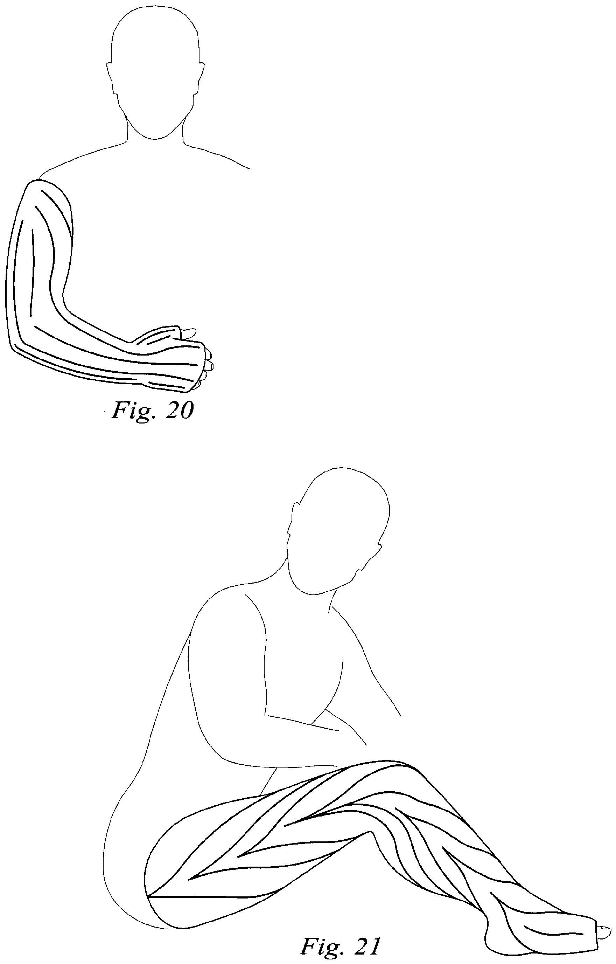

Figs. 20 and 21 show liners with channeling which approximately follows the lymphatic flow along the limb. Such compression garments are well known in the art and made of chipped or cut foam and sold under the name Med-Telesto, Solaris, and Jovi. The garment may contain foam of different densities. Higher density foam is thought to provide more aggressive reduction of underlying lymphedema and fibrotic areas from a process known as lipodermatosclerosis. In this disclosure, the author discloses a liner which has padding made of spacer fabric material. This material has quite an advantage over foam with lower weight and greater breathability. The spacer fabric material may be cut into strips and sewn in place, or generated as a sheet or tubing or padding to specific areas as outlined in Fig. 21. In another embodiment, the spacer fabric is cut into squares or shredded in a manner similar to a foam shredder and used in the garment instead of foam. The use of spacer fabric is possibly quite important, as it may make the garment more breathable, dry quicker, less weight, lower profile, and decrease skin moisture which could therefore decrease risk of dermatitis and fungal infections and cellulitis compared to similar products using closed or open cell foam.

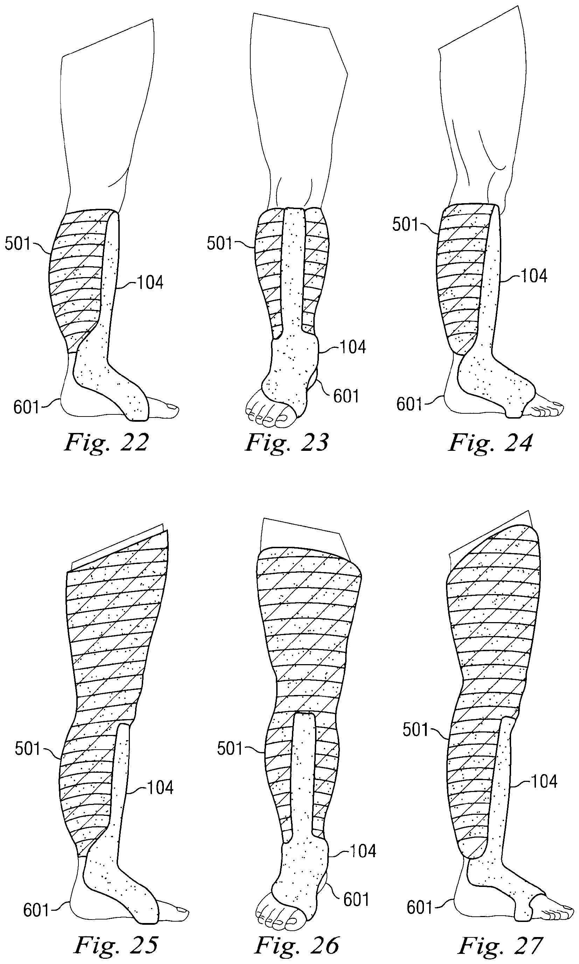

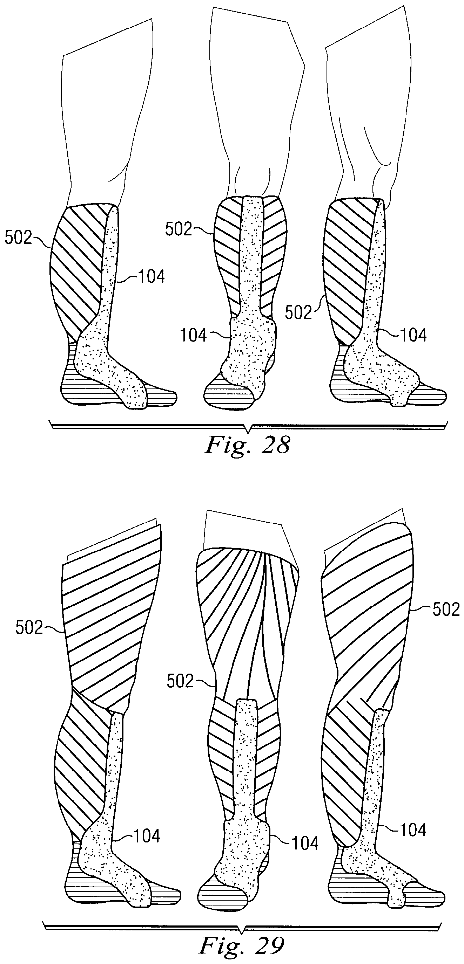

Figs. 22 to 24 show different liners employing the materials and arrangements disclosed herein. Figs. 22 - Fig.24 show a liner shape 100 with a first padded region 104, a second padded region 501, and a third unpadded or less padded region 601. The third region 601 may comprise, for example, the liner material without padding. The first and second padded regions 104, 501 may be formed to have any of the features discussed with respect to other embodiments. Here, the padded region 501 may include a different level of padding than the region 104, providing different levels of compression when used with a compression garment. Fig. 28 and 29 show a second padded region 50 2 having the channels discussed above with reference to Figs. 20 and 21. Figs. 25-27 and 29 show a thigh-high liner with features similar to those discussed with reference to Figs. 22-24.

As discussed above, the padded regions of the liners may be formed by incorporating a padding material formed of a spacer fabric therein. Conventional padding materials, typically comprising foam, gel, or other padding materials provide only limited breathability, sometimes resulting in heat rash dermatitis, fungal infection, itching, and overall patient discomfort. This can also result in lack of patient compliance when such liners are used and prescribed. In short, this problem with conventional materials can be detrimental to attempts at edema reduction and healing of wounds in edematous limbs.

However, exchanging conventional padding materials with spacer materials eliminates many of the shortcoming of the conventional materials. As stated above, spacer material comprises a layered fabric material formed of at least two layers of materials separated by threads or filaments extending from one layer to the other to compressably maintain the spacing in a way that provides cushioning to loading on one of the layers. Because the spacer fabric has mostly air in-between the two layers, the material is highly breathable. Furthermore, the layers also may be highly breathable. This higher breathability means less incidence of heat rash dermatitis and less risk fungal infection. This reduction in the incidence of dermatitis, itching, and overall patient discomfort directly addresses issues contributing to lack of compliance with compression in patients. Accordingly, incorporating spacer fabric as a padding material would increase patient compliance, directly affecting and improving the results of edema reduction and healing of wounds in edematous limbs. The spacer fabric padding may be used as sheeting cut to fit the area that needs padding, may be cut and sold in shapes (ankle pad, shin pad, mastectomy pad, oval shaped pad), and may be used over fibrotic and indurated areas to soften tissue and help reduce lipodermatosclerotic changes. Alternatively, the spacer fabric padding disclosed herein may be incorporated into a liner product. The spacer fabric may be woven with different monofilament densities of sizes even within a sheet, in order to provide softer padding to some areas and harder padding to other areas. Since some spacer fabrics are woven on a circular knit machine, the machine may be programmed to create padded liners directly with an automated or semiautomated process, either with or without channeling. Such a product has significant cost advantages, and would likely weigh less than comparable foam liners and have greater breathability.

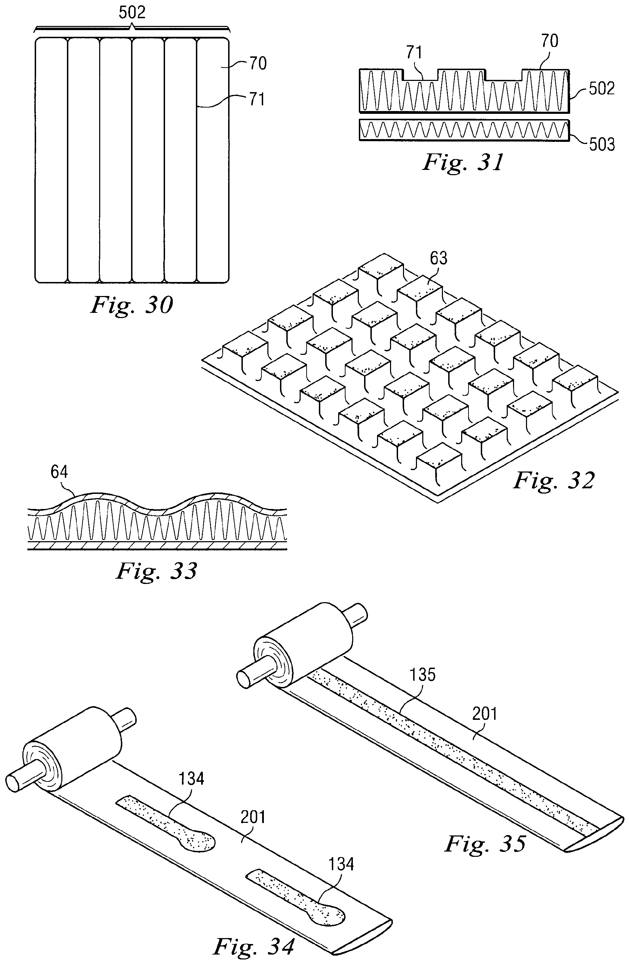

Figs. 30 and 31 show an exemplary spacer fabric 502 usable as a part of the liners disclosed herein. Here, the fabric is sold as a sheet or roll. In this embodiment, the fabric includes linear grooves 71 which are cut or made during manufacturing. These padded and grooved areas provide low and high areas of compression. Such a spacer fabric has never been disclosed. The spacer fabric material has inherent qualities or higher breathability, lower weight, and increased comfort. By varying the length and size of the filaments extending between the two layers, different densities can be created. The grooves 71 and the padded areas 70 may vary in width and height depending on the application. In some applications, a second layer of spacer fabric material or padding 503 also may be included.

Fig. 32 shows another embodiment of the liner padding as spacer fabric material. This time instead of channels the foam is made with taller areas of compression 63 which are square or pyramid shaped with or without tapered sides. In use as a part of a liner with a compression garment, the taller areas are placed in contact with the patient's skin. Then under compression from the compression garment, the taller areas 63 move around under the garment against the skin and the movement is thought to facilitate the breaking up of fibrotic areas of tissue and help soften the tissue over time. Fig. 33 shows another embodiment cross-section with sloped sides to the raised squares 64. It is understood that other embodiments and shaped areas are possible.

Figs.34 and 35 show a liner roll comprising a plurality of connected liners manufactured to have both the padded region and the less padded regions built in. In some embodiments, the product may come in a large continuous roll such as in Fig. 34, which can be cut for each patient. In this embodiment, the padding of the first region 134 provides pressure relief to the anterior tibial crest and anterior ankle areas. This has use in wound care, physical therapy, and orthopedic offices to provide protection under compression bandages, wraps, orthotics, and casts. Such an embodiment may be preferable for hospital and clinic setting to a single liner, and may be more cost effective.

In another embodiment as shown in Fig. 35, the padding 135 is wide enough that it provides relief to the anterior ankle, anterior tibial crest and dorsal foot and comes in a continuous roll. It is understood that it may have other uses too, such as padding for the inside or outside of an arm.

In some embodiments where the padding material chosen is foam, the density chosen may reflect the application. It may be closed cell so it does not retain moisture easily, or open cell foam for wound care application or other reason. The foam may be reticulated for better breathability. If foam is used, it may be laminated on one side so that foam is not exposed. The foam may be on the inside or outside of the liner.

In some embodiments, the padding may contain multiple holes in it to improve breathability. These holes may also affect the skin surface pressures under the device. The size and location of such holes may be changed to improve breathability, skin surface pressures, and comfort with joint range of motion, depending on the application. For instance, in one embodiment it may be desirable to use padding with high hole density or large holes over the anterior ankle area for better breathability, but still use thicker foam or spacer fabric material to better protect the tibialis tendon. It is understandable to one known in the art that other areas of high flexion such as the elbow and knee may desire more strategic hole placement or size to provide optimal padding, breathability, and stretchability to the affected area of the limb. The padding in some embodiments would have multiple small holes poked in it to allow for breathing or with dye cut patterns congruent at edges, yet to allow motion at joints and/or to allow for breathing of the liner.

The liner may be single use, a limited re-usable, or re-usable product with long life span.

In some embodiments the liner / padded areas would incorporate antimicrobial materials/chemicals/products into liner material to reduce pathogen colonization and reduce risk of infection.

In some embodiments, there would be areas of the liner with padding to protect bony areas, and other areas with channeling to help direct lymph flow and facilitate oedema reduction.

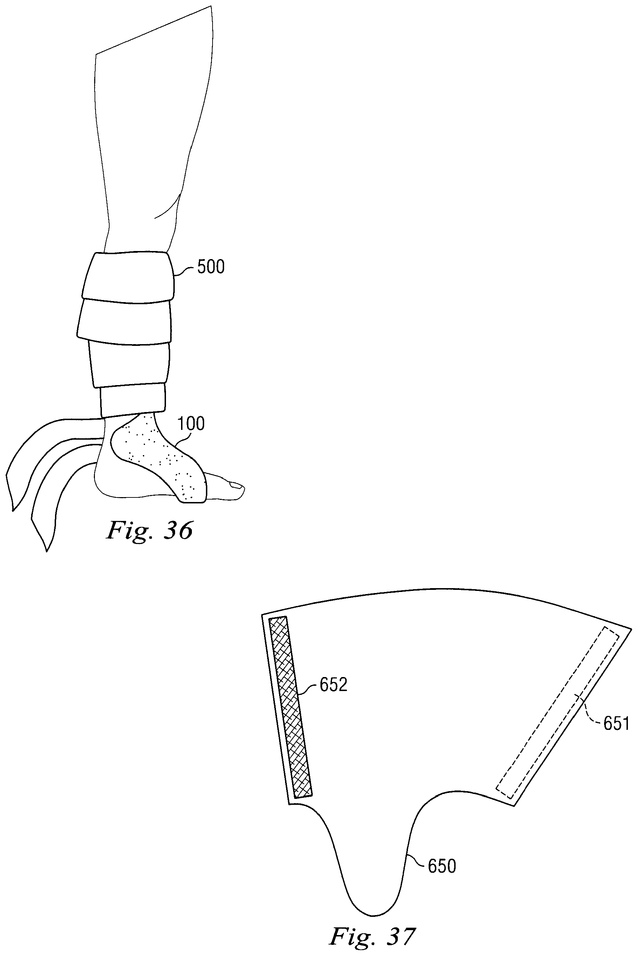

Fig. 36 shows one example of a system for treating edema or other condition. The system comprises a compression garment 500 usable with the padded liner 100. After the padded liner is donned and the first padded region is sufficiently disposed to protect the hard tissue areas of the patient in the desired manner, the compression garment 500 is wrapped about the exterior portion of the liner to provide compression therapy to the limb. The padding increases patient comfort in the hard tissue areas, while the lack of padding in the softer tissue areas provides breathability, is cooler, light-weight, and overall less bulky than a fully padded liner. In some embodiments, the compression garment 500 is a garment having bands having short-stretch properties and may be any of the garments disclosed in U.S.

Patent Application No. 10/975,590 filed October 28, 2004 and U.S. Patent Application No. 11/733991 filed April 11, 2007.

Using garments having the properties of short-stretch provides many benefits to a patient. A user can stretch the band and see and/or feel when the maximal stretch level is reached because the band may have a limited stretch range. Using this, the user can 'dial into' the correct level of compression when applying the garment, without needing to use a pressure sensor or an indices-type system to determine the correct compression level. This provides a very simple, but very reliable, method of reproducing the correct level of compression every time the garment is donned.

Because the bands are applied at or near maximal stretch, it will not stretch further. Therefore, the garment provides maximal augmentation of the calf muscle pump and the more the leg tries to swell, the more the garment will work to prevent swelling.

Thus, such a short-stretch garment has a high static stiffness index, which has been suggested to measure efficiency of the bandage / garment on the augmentation of the calf muscle pump.

The garment can be designed to be a single use disposable device, or can be designed to be reusable. For severe venous ulcerations with lots of drainage or bioburden, the garment can be designed to be of disposable materials similar to those used in multilayer compression wraps. For mild draining ulcerations, the garment can be designed to be re-usable. In some embodiments, the garment can be used to heal the ulceration, and then the user can continue using the garment for maintenance compression in order to prevent recurrence.

The garment can also be designed to provide graduated compression. For a typical 30-40 mmHg compression stocking, for example, there can be 30-40 mmHg compression at the ankle, but perhaps 20-30 mmHg at the calf level, 15-20 mmHg in the distal thigh, and 8-15 mmHg in the proximal thigh. Graduated compression provides more compression distally on the limb than proximally, and compensates for gravity to provide optimum compression levels. Different embodiments of the garment can include one of the features listed below to provide a garment with graduated compression. The features listed below can also be mixed and matched to provide graduated compression by combining these various modalities in combination to provide a sophisticated garment with various compression levels.

Spacer Fabric as a Compression Garment

In some embodiments, the Spacer Fabric may be used to make not only a liner for use with a compression garment, but may be used to make a compression garment itself. The spacing and filaments between the layers provides cushioning or air padding. Thus, in this embodiment, the garment itself is a padded compression garment. In one embodiment, the Spacer Fabric would be generated to create a short-stretch range of compression 15-90% maximum stretch with resting compression at end stretch of 15-20mmHg, preferably with fairly abrupt end-stretch so that the user can feel when the band no longer stretches. The outer surface of the Spacer Fabric can be generated to have Velcro®-type hook compatibility, and the opposing bands can be secured in place with standard hook material. This garment would ideally have relatively abrupt end-stretch or bandage lock-out so that the user can readily identify when the garment is applied at maximum stretch. This embodiment may be excellent for postop surgical use and hospital use to prevent DVTs in patients, for example. Examples of compression garments that may be formed with bands of spacer fabric are any of the garments disclosed in U.S. Patent Application No. 10/975,590 filed October 28, 2004 and U.S. Patent Application No. 11/733991 filed April 11, 2007. Other embodiments may have 8-40mm compression when applied at end-stretch to a limb portion.

In another embodiment, the spacer fabric would be laminated to a woven and / or knitted stretch or compression fabric or UBL (unbroken loop fabric) to provide therapeutic compression levels. In one embodiment, the garment would consist of Spacer Fabric with UBL on the outside of the garment. In another embodiment, there is a woven fabric which provides the end-stretch on the inner layer, a spacer fabric to provide padding, and an outer layer of UBL material. Other embodiments are possible, depending on materials and properties chosen. The compression fabric could be knitted of Rochelle or Tricot or another knitted weave, or could be a woven fabric. The laminated fabric could provide the compression, the bandage lockout or a combination of both, such that the combined layers provided therapeutic resting compression level of 8-50mmHg at or near end-stretch when applied to a limb at rest, and a maximum elasticity of 15-90% maximum stretch with preferred range of 25-50% maximal stretch. Additionally, the laminated woven or knitted fabric could provide the Velcro®-like hook compatible surface, as is known in the art. The fabric(s) could be located on the inside or the outside of the spacer fabric or both. The combined result would be correct compression level and built in padding to prevent injury to the limb. Woven fabrics are especially valuable in designing such a system due to their ability to be designed with abrupt end-stretch.

In use, the system with a liner and a compression garment as disclosed herein may be used to treat a swelling condition such as edema. The liner is first applied to the limb to be treated. The liner may include the first and second regions discussed herein, with the first region having thicker padding than a second region. In some embodiments, the liner is completely formed, with the padded first and second regions prior to being worn by the patient. Thus, the liner is applied while in a completely assembled state. Applying the liner may include pulling the tubular liner from the proximal end over the foot in the manner of donning a sock or may include forming the liner on the leg. The liner may be form-fitting about the limb and may be adjusted or aligned so that the padded first region overlies a bony area on the limb as desired. In some embodiments, the padded liner is shaped to overlie specific portions of the limb to provide the padding to the bony area. In some examples, the padded first region may be applied to overlie at least one of the anterior tibial crest, anterior ankle, malleoli, and the first and fifth metatarsals. In some examples, the liner may be applied so that the padded first region overlies a plurality of any of the regions discussed. In addition, applying the liner to the limb also includes aligning the less-padded second region over soft tissue on the limb. In some embodiments, the less padded second region is unpadded and comprises only a thin layer of liner material, permitting the liner to be lightweight, breathable, and comfortable. In some embodiments, the padded first region employs spacer fabric having first and second fabric-type layers held apart by filaments such that a layer of air lies between the first and second fabric-type layers.

Once the liner is properly applied with the first and second regions in place as desired, a compression garment may be donned over the liner on the limb. The compression garment may comprise a series of connected bands wrappable about the limb and connectable to each other to secure the garment on the limb. In some embodiments, the garment is formed to have properties of a short-stretch material to apply compression to the limb. The padding in the liner distributes compression loading about the bony areas in a manner that makes wearing the compression garment and liner relatively comfortable for the patient. In some examples, the method of use also includes cutting the liner from a liner roll before the liner is worn by the patient.

Fig. 37 shows another embodiment of the padded liner. This embodiment shows the liner made incorporating padding for the entire limb, such as spacer fabric. In this embodiment, the spacer fabric is cut so that it can be wrapped around the entire lower limb. In the preferred embodiment, the outer portion of the garment is Velcro®-like hook compatible or has hook compatible portion 652, such that attachment mechanism 651, made of Velcro®-like hook material, may be used to attach to the attachment mechanism 651 in an overlapping manner. The tongue area 650 is designed to provide padding to the anterior ankle area and dorsal foot. While this padding design is bulkier than some other embodiments, the spacer fabric design is highly breathable and provides adequate padding to protect the underlying tissues. Such a garment may be desirable for very fragile skin or skin with wounds circumferentially, for example.

Although disclosed as being used over the foot and leg, it is also contemplated that the liner disclosed herein may also be used to treat the arm, shoulder, hand, wrist, knee or other portion of a limb on a patient or an animal. In addition to being used under a compression garment, it also may be used under an orthotic, cast, or bandage. The padding of the second region may be located for example, to provide increased padding over the anterior tibial crest, the anterior ankle, over and around the medial and lateral malleoli, over the $1^{st}$ metatarsal head medially and the $5^{th}$ metatarsal laterally, over the dorsal foot, and/or over the knee area for example. It also may be used to provide increased padding over the palmar hand, the dorsal hand, the wrist area, and/or the elbow, for example. In some embodiments, the padding is spacer fabric while in other embodiments, the padding is chipped and/or channeled foam mixed with smooth foam over the aforementioned areas. The liner may have therapeutic compression properties in the range of 8-50mmHg resting compression.

**Trim-To-Fit Compression Garment**

Correct fitting of compression garments to limb shapes necessitates custom and off-the-shelf varieties. Foot length varies considerably, and there is variation in width and therefore circumference of feet, as well as considerably variability in ankle and calf circumferences and shapes, as well as height. The same applies for thigh high and arm and hand sizes. Most compression garment manufacturers, therefore, use custom and off-the-shelf solutions to fit a wide range of patient sizes. The custom garments, however, take time to manufacture. It is not uncommon, for instance, for custom compression stockings to take one month or longer from date of order until the patient receives the garment. Furthermore, errors in manufacturing and measurement sometimes necessitate remanufacturing the garment or altering the garment in order to get a proper fit. This is very inconvenient for the patient, who needs therapeutic compression immediately, and must make-do with an off-the-shelf garment or bandage until the custom garment arrives and fits correctly.

Furthermore, in some patients you need compression over the distal forefoot, at the level of the metatarsal heads, in order to prevent lymphostatic pooling of edema in this area. In other cases, you want to avoid compression over the metatarsals in order to reduce chance of injury due to pressure directly over a bony surface. The ability to make these clinical decisions and take immediate steps to provide a correctly sized and suitable garment is very important in order to provide proper therapeutic compression safely and effectively in a patient.

Lastly, patient limbs vary in geometry from conular to tubular to other odd-shaped morphologies. At present, when a band is wrapped horizontally about a limb that is conular, it is tighter around the wider, more proximal portion while being looser around the distal, more narrow portion. The net effect of this is to promote the likelihood of the band sliding down the limb, which becomes more problematic on more conular-shaped limbs.

What is needed, therefore, is a garment system that is customizable at point of sale to patients by a medical equipment company, clinic, hospital on site, or even by the patient themselves so that the garment is immediately available and correct fit can be established with on-site customization that is simple, reliable, and predictable. One way of customizing as described uses a trim-to-fit solution permitting providers and even patients to trim the garments to provide a proper size while also providing proper and desired compression levels.

For the trim-to fit solutions, the material would ideally be short-stretch material or a material having properties of short stretch, with maximum elasticity of 15-100% such that the garment provides a compression level that falls within the range of 8-50mmHg, when applied on a resting supine limb at or near end-stretch. It is understood however, that non-elastic or moderate-stretch or long-stretch embodiments may be chosen. A variety of standard materials known in the art for treating swollen limbs may be used for this invention. It is understood that the appropriate choice and location of markings would take into account the stretch of the material in order to properly trim and fit the garment to the patient.

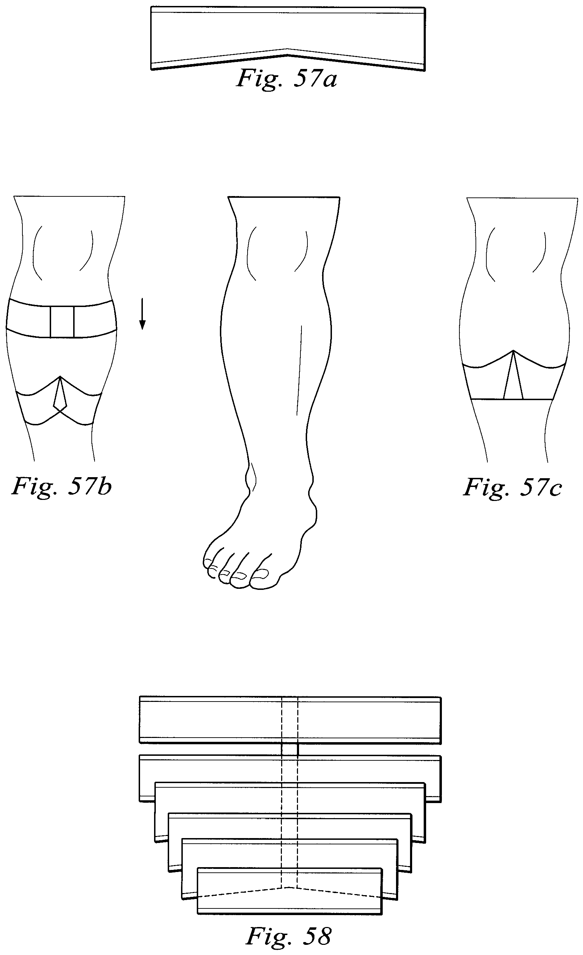

Additionally, for use over legs, thighs, and some arms that are more conular-shaped, it would be beneficial for a few of the bands to be trapezoidal or trapezoidal-like in nature, with both band ends wider on the distal side of each band such that as the band is wrapped around a conular-shaped limb the bottom edge of the band may be applied in a more horizontal plane. This will provide more even compression to the limb and will better resist sliding down the limb. For one preferred embodiment, a 1 way stretch material may be preferred in order to maximizing working compression and minimize band narrowing as it is pulled. In order for the garment to fit best, however, it may be desirable for the material to stretch in the width of the bands at least 5-25%, depending on the angles of the trapezoidal or trapezoidal-like shape. In some embodiments with these trapezoidal shaped bands, the bands may have horizontal shape proximally, but trapezoidal shaped distally with less width to the band in the middle than at the ends. For such a band, if it is pulled around a conical shaped limb portion, the result would be a more uniform distribution of compression and more upward pulling vector force, which would prevent garment slippage over time. One example of such a trapezoidal band is shown in Fig. 57a and an example of a garment using such bands, along with straight bands, is shown in Fig. 58. Fig. 57b shows application of a rectangular shaped band on a conical shaped limb. In this case, the compression is largest at the top of the band and the bottom portion of the band has less compression, making it more prone to slip. As shown in Fig. 57b, the band slips around the sides and the central portion tends to hold its position more. Fig. 57c shows a trapezoidal shaped band applied to the limb. Since the band is pulled tighter at the base, it conforms better to a conical shaped limb and is less prone to slippage. Fig. 58 shows a garment where some of the bands are trapezoidal shaped. In other embodiments, all or just one of the bands would be trapezoidal shaped.

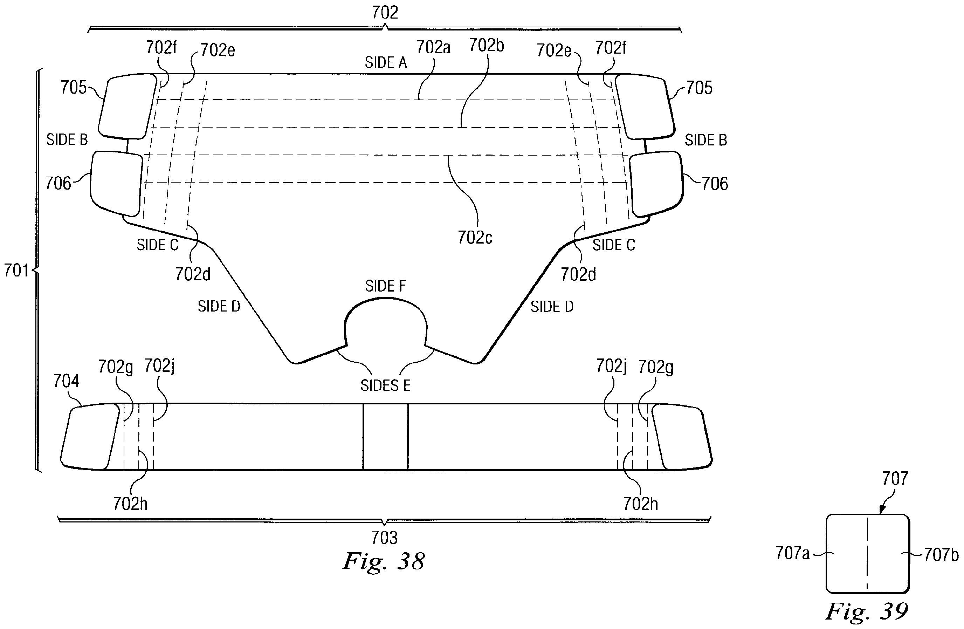

Fig. 38 shows embodiments of such a garment for the lower limb. It is understandable that a similar design can be used for thigh high or arm garments as well as other limbs or body parts of a person or animal. Fig. 38 shows one preferred embodiment of a footpiece. This footpiece 701 has lower portion 702 and ankle portion 703. The lower portion 702 has markings showing appropriate places where the footpiece can be trimmed in order to properly fit a wider range of feet. The markings 702a - 702c show where to cut the length of the footpiece. The lines 702d - 702f show where to trim to adjust the width of the footpiece. The sides E are sewn together in the preferred embodiment and attached to the middle of the band 703.

The band 703 has markings 702g-702j which show where the band may be trimmed in order to better fit the size of the foot. The band 703 is designed to be trimmed on both sides along the same marking, in order to fit symmetrically, but may be trimmed as needed to properly fit the limb portion. The band 703 is designed to be applied around the ankle and downward across the dorsum of the foot, but may be applied directly across the ankle if desired.

Similarly, the bottom of the footpiece 702 is designed to be trimmed on both sides of the markings 702d-702f in order to symmetrically fit the foot, but may be trimmed differently as desired in order to properly fit the limb portion. Attachment methods as are known in the art may be used to attach the pieces together, as is known in the art. In the case of the preferred embodiment, pieces 704 - 706 may represent the attachment mechanism. In one preferred embodiment, the attachment mechanism would be a piece of Velcro®-like hook material. In other embodiments, the attachment mechanism may be tape, material with fabric glue, button, metal clasps, holes for laces, or other mechanisms as are known in the art.

In this preferred embodiment, the garment would have an outer surface with areas of Velcro®-like hook compatible loop material in order to fasten to the hook material. In this embodiment, the attachment mechanisms 704-706 are selectively detachable.

In other embodiments, the attachment mechanism may be manufactured differently. Figure 39 shows close up of another embodiment of the attachment mechanism. In this embodiment, the attachment mechanism has an area of semi-permanent or permanent mechanism 707a, such as industrial strength Velcro®-like hook and loop or very strong adhesive. Once the garment was trimmed to fit the limb correctly, the attachment mechanism 707a would then be fastened to the end of the band. Once attached, the area 707a would be difficult to remove. A second area of the attachment mechanism 707b, is designed to be less permanent and easier to remove. This way the garment could be removed periodically or daily at area 707b, while area 707a stayed attached. In some embodiments, this would make the garment easier to don and doff and provide a simpler and more reliable solution, which may be less confusing for the person donning and doffing the garment. In other embodiments, the garment may be single use and the attachment mechanisms may be quite permanent, requiring cutting away of the garment or destroying the attachment mechanism upon removal. Such an embodiment may be preferable for a single use or limited reusable embodiment of the garment, such as for wound care, clinic or hospital use.

For attachment mechanisms 704, it may be preferred to have the attachment mechanism such as Velcro®-like hook material on both sides. For the footpiece attachment mechanisms 705 and 706, however, there may only need to be attachment mechanism on one side. For example, once the garment was trimmed along lines 702d on both sides, one side of the material would fold under the other side of the material to create a small amount of overlapping. This would be necessary and desirable in most embodiments in order to prevent windowing (area where there is no compression over a limb portion). In this case, the attachment mechanism may only need to be on one side, such that the attachment mechanism holds the sides together once applied over the limb portion.

There are additional benefits to the design of an attachment mechanism which can be placed on either the inside or outside portion of the limb. For instance, the attachment mechanism may be best applied on the inside or the outside portion of a foot. Since the attachment mechanism is removable, a single design can provide right and left foot sizes and provide symmetry. This would reduce the number of garments needed to properly stock and fit a wide range of sizes, and provide better patient comfort and fit with more symmetrical appearance.

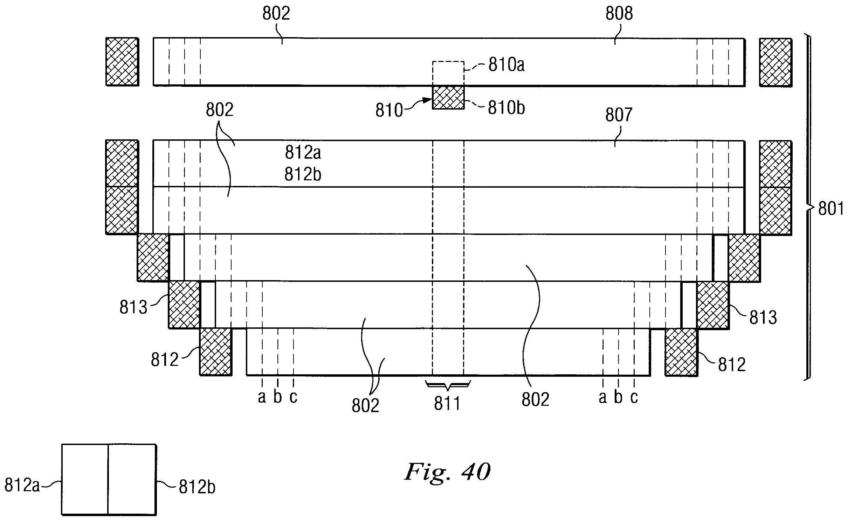

Fig. 40 shows one embodiment of a legpiece 801 of the present invention using trim-to-fit design. In this embodiment, one or more of the plurality of bands 802 have markings 802a-802c which show where to trim the band in order to fit a smaller size limb. By determining limb size and where to trim each band, the garment can fit a wider range of patients. In addition, it can be used for active treatment phase edema reduction as the garment size may be reduced as the edema reduces allowing the patient to be treated and released in the same garment creating great cost savings for both the patient and health system.

Since leg length varies considerably among patients, band 808 has attachment mechanism 810 to attach to the upper or lower bands or an optional spine 811. The attachment mechanism 810 has sections 810a and 810b. Section 810a attaches to the more proximal band 808 on either the inside or outside of the band. The section 810a may extend the entire width of the band, creating a spine over this single band. Attachment mechanism 810b attaches to the inside or outside of band 807. The attachment mechanisms may be of any method as known in the art, such as permanently, semi-permanently, or selectively detachable. In one embodiment, the attachment mechanism 810a is sewn onto the band 808 and the attachment mechanism 810b is sewn onto the spine 811. The user can then cut away the upper band 808 if desired by trimming the attachment mechanism 810 between sections 810a and 810b to shorten the garment. More than one attachment mechanisms 810 can be used, with one between each band. This allows the garment to be trimmed to shorten it in an easy and reliable manner in order to better fit the patient.

By creating each garment with the upper band(s) as separately attachable and/or trimmable, there is no reason to stock short, regular, or tall garments, reducing inventory and increasing the likelihood that the trim-to-fit garment can fit any given patient population. This is especially important for Durable Medical Equipment companies, hospitals, and clinics, who would prefer to stock fewer sizes to fit their range of patients. By reducing inventory, cost savings are realized. By having customizable features, the garment can better fit any given patient.

Attachment mechanism 812 is used to connect the ends of band 802. The attachment mechanism 812 may start on the outside of the overlapping band on either side. This is advantageous, as the trim-to-fit legpiece can therefore be made such that the attachment attaches to the inside or to the outside (medial or lateral side) of the legpiece, depending on patient ability and preference. In most embodiments, the patient will want a Velcro®-like attachment mechanism that is utilized such that it is on the outside of the legpiece so the Velcro®-like attachment mechanism does not rub against each other medially on the limb.

The attachment mechanism 812 may have sections 812a and 812b. One of the sections may be permanent or detachable but with greater holding strength than the opposite end. For example, if section 812a of the attachment mechanism used standard Velcro®-like attachments, and section 812b used more industrial strength Velcro®-like attachments, then section 812b would be designed to be applied to the end of the band. The user would then overlap the bands appropriately and use section 812a during daily use to don and doff the garment, leaving section 812b attached. Section 812b could be removed, however, for instances like washing the garment in a washing machine, where the Velcro®-like hook and loop could increase wear or cause garment entanglement which would make garment less easy to clean and use on daily basis.

One example of a preferred embodiment of the attachment mechanism uses HiTex corporation HTH-833 as section 812b, and HitTex corporation trihook-150 BEI as section 812a. Because HTH-833 is very aggressive hook material and very difficult to remove, it would be designed to stay attached to the end of band 802 long term. The patient would then remove and apply section 812a during daily use. The entire attachment mechanism 812 could be removed during weekly washing and on an as needed basis, and the rest of the time the patient would wash the liner daily with the compression garment.

Another benefit of attachment mechanism 810, is that the degree of overlap of one band 802 relative to one or more other bands 802 is realized. In some embodiments, there will be an attachment mechanism 810 between each band, such that some bands may overlap completely, and other bands may overlap less. By determining the compression of each band, and determining the desired compression level to that limb portion, one could change the degree of overlap over certain portions of the limb to increase compression. For instance, most compression garments have gradient compression with more compression at the ankle and less compression proximally. By overlapping bands more distally near the ankle and less proximally near the knee area, one can create gradient compression. By applying the garment at end-stretch, you can create graduated end-stretch compression if desired. Furthermore, by selecting bands with more compression at end-stretch or less compression at end-stretch, you can select more or less compression to a limb area. Thus the trim-to-fit allows the user to be able to customize compression levels to desired limb portions.

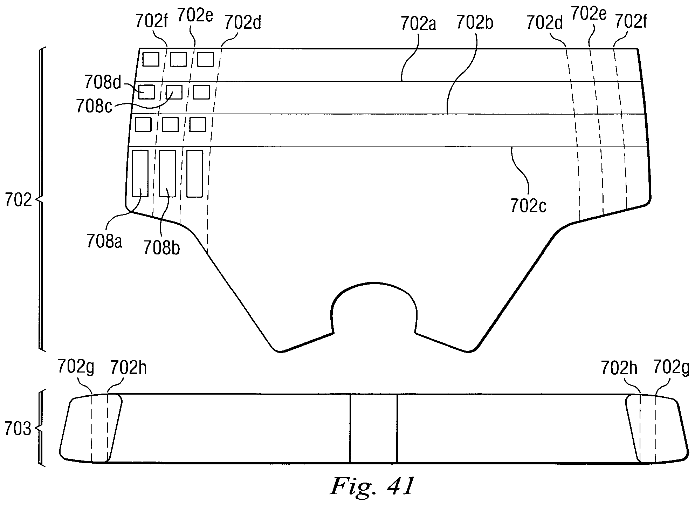

Figure 41 shows another embodiment of the current invention, which shows multiple attachment mechanisms 708. In this embodiment, the attachment mechanism is already in place on the garment 702. In one embodiment, the attachment mechanisms 708 represent pieces of Velcro®-like materials that are permanently or semi-permanently attached using a method that is known in the art such as sewing, RF welding, gluing, or ultrasonically welding into place. The attachment mechanism is divided between the markings on the garment, in order to facilitate easier trimming of the garment to properly fit the underlying limb portion.

In another embodiment, attachment mechanism 708 may be one large piece with the markings on the attachment mechanism and mechanism of attachment as shown on the band 702 in Fig. 41. Here, the garment can be safely cut to size as desired along the lines 702E or 702F even through the attachment mechanism, and still work properly and reliably.

The markings on any of the garments in Figs. 38-41 may be color coded, may have indicia, labels, or other identifying marks that help identify or determine which line the garment should be trimmed in order to provide the proper fit. In some embodiments, the markings are woven into the fabric, while in other embodiments, the markings are applied using an ink or dye. Furthermore, the markings may be permanent or may wash or wipe off, such that after customization to fit, the markings may be removed. In some cases, a measurement form or table may be used to help guide the determination and help trim the garment quickly and effectively without error in order to provide proper fit and no markings would be needed. Guide patterns that overlay the garment and identify the trim lines also may be used. Other markings are also contemplated.

Joint Piece

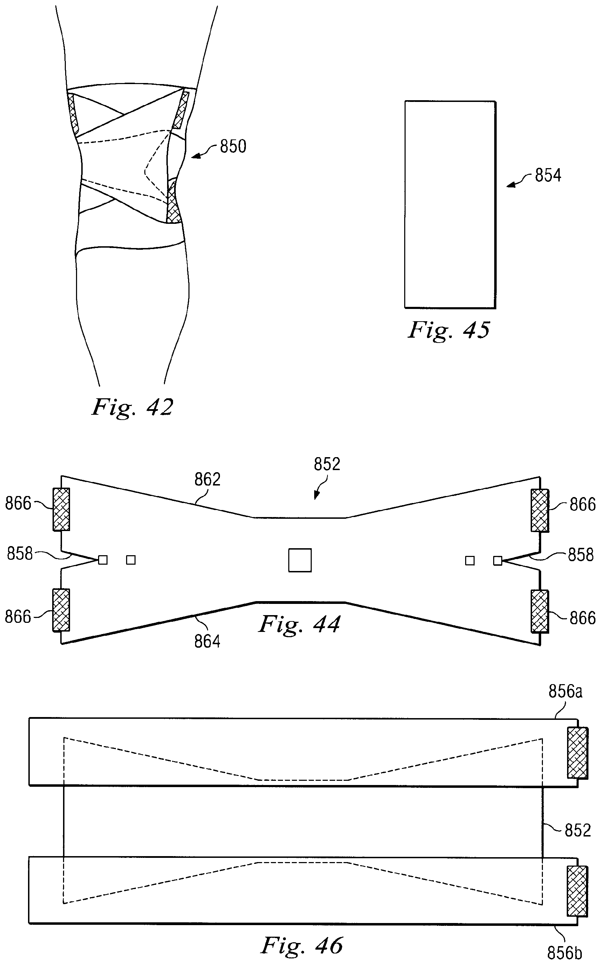

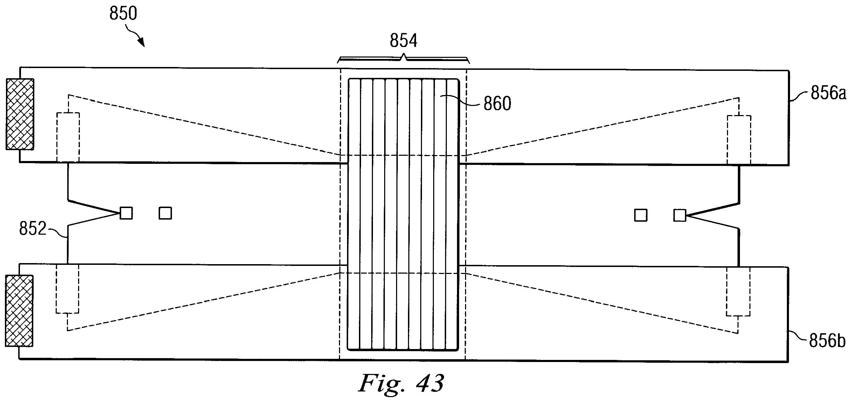

Fig. 42 shows an embodiment of a complete kneepiece 850 applied to a knee joint as seen from the front of the knee. Fig. 43 shows the kneepiece 850 lying flat. Components making up the kneepiece 850 of Fig. 42 are detailed in Figs. 43-46. These include an hour-glass shaped middle band 852, an optional spine 854, and two straight bands 856. Figs. 43 and 44 show the hourglass shaped middle band 852 with notches 858 cut out as seen at the top and bottom of the hourglass shape. The hourglass shape band 852 interposed between the optional spine 854 and the straight bands 856 allows the kneepiece 850 to conform to the knee while providing good compression at the same time.

Padding 860 is added to the posterior of the kneepiece 850 as shown in Fig. 43. This padding 860 helps protect the sensitive arteries, veins, and nerves that course along the posterior of the leg when the knee is bent, protecting these structures from an impinging garment during flexion. It also increases the central diameter of the kneepiece 850 and provides some rigidity to the design, both of which help prevent the kneepiece from wadding from both the bottom and top towards the center after repeated flexion and extensions of the knee. Because of the benefits it provides, the padding 860 can be an important component.

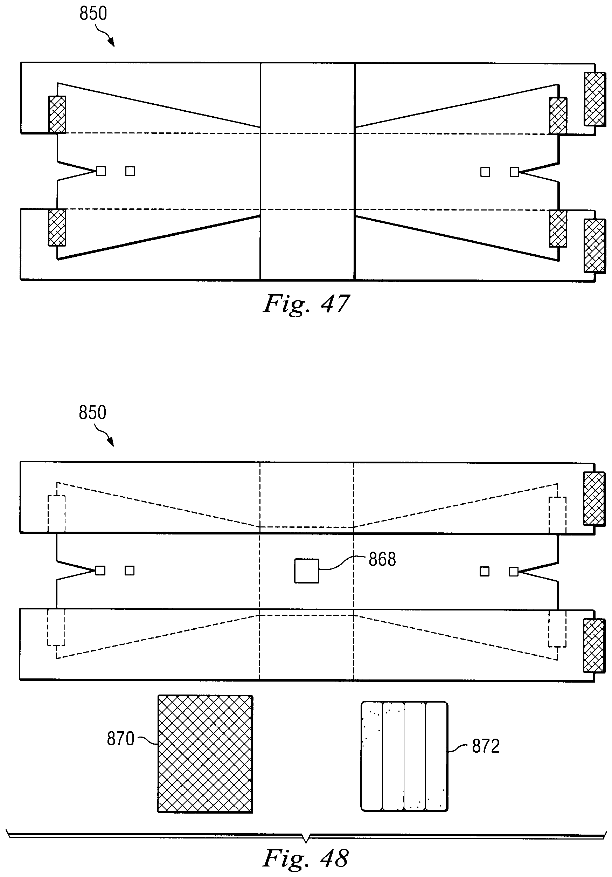

In some embodiments, the kneepiece is made with and without the proximal straight band 856a and the distal straight band 856b, leaving only the middleband 852, the optional spine 854, and the optional padding 860. In these embodiments, the kneepiece could be directly attached to a separate thighpiece. Alternatively, a Velcro®-like material strip (not shown) running horizontally along the proximal or upper lateral portion 862 and along the distal or lower lateral portion 864 could be employed to hold the kneepiece in place on the thighpiece and legpiece respectively, rendering the kneepiece completely detachable. Fig. 47 is a back view of the kneepiece 850 shown in Fig. 43.

This customizable design, and the option to use or not use the straight bands 586 with the middle band 852, allow for user preference in placement, allow the user to use the legpiece with or without both the kneepiece and thighpiece, and allow the kneepiece to be used by itself, such as for post op knee surgery. The design allows the kneepiece to be quickly and easily removed by the doctor for dressing changes and reapplied similarly. It could also be used for sport injuries. In addition, in some embodiments, the kneepiece employs channeled foam or spacer type fabric liners and facilitates edema reduction. The channeled foam may be longitudinally directed to facilitate fluid removal, while providing padding for comfort and protection to the posterior knee area.

The Velcro®-like hook materials could either be attached to the band ends or made detachable so that the kneepiece too could be a trim-to-fit device. In some embodiments, the bands, including the spine are held together by fasteners, such as the hook and loop fasteners 866. These fasteners 866 can be used to connect the middle band 852 to the straight bands 856, and likewise, similar fasteners may be used to connect the padding to the middle band 852 or spine 854

Additionally, the bands and/or spine may be made from both one way stretch and two way stretch. The two way stretch bands provide a softer end stretch over more sensitive areas such as joints, and allows for range of motion in addition to compression when used over joints. Thus, two way stretch bands may be preferable for use over joint articulation areas, whereas one-way bands may be preferable over non-joint areas, as they are less prone to necking (shrinking in the middle when stretched).

Some embodiments, such as the kneepiece shown in Fig. 48, include a mesh pocket 870 at the narrow portion of the hourglass middle band 564 which locates at the back of the knee when worn. This pocket 870 provides for insertion and removal of selected padding pieces 872, such as a foam insert, for better protection of sensitive parts of the back of the knee.

The pocket 870 and padding 872 may be selectively or permanently attached to a central attachment mechanism 868 on the middle band 852. This central mechanism may be a hook and loop fastener, for example, but also may be any connection mechanism, including an adhesive, sewing, ultrasonic welding, and others. In some embodiments, the pocket is permanently attached to the kneepiece 850 and the foam may be removed fro the pocket for replacement or cleaning.

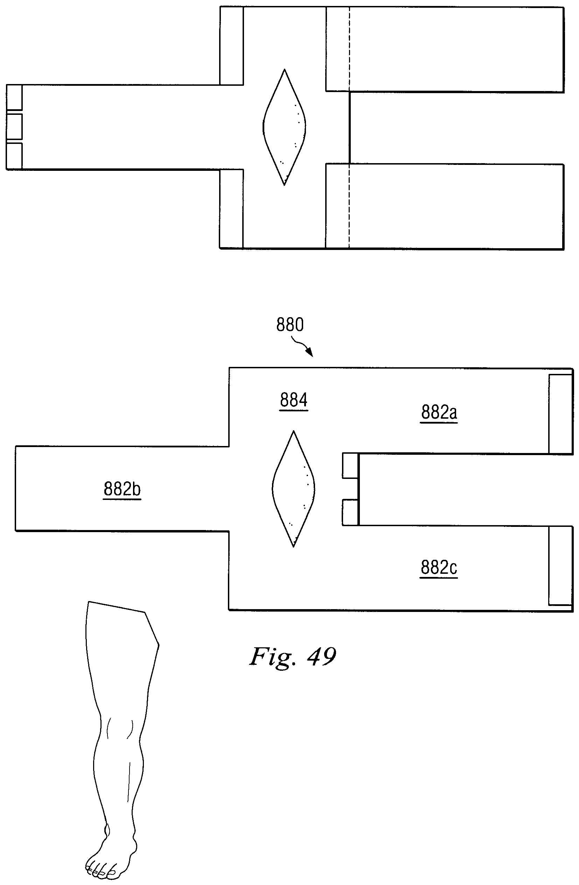

Figs. 49-52 show additional embodiments of kneepiece systems. Fig. 49 shows a kneepiece 880 having three extending straps 882a-c. These straps connect to each other along a central spine 884. In some embodiments, the bands 882 and spine 884 are integral, and may be cut or stamped from the same material. In other embodiments, the bands are attached to each other along lateral edges, thereby forming the spine 884. In yet other embodiments, the garment is formed of a single piece. Their construction uses band materials similar to those used in figs. 42-45.

The oval and round portions are locations of stretch material to provide greater comfort for the kneecap area.

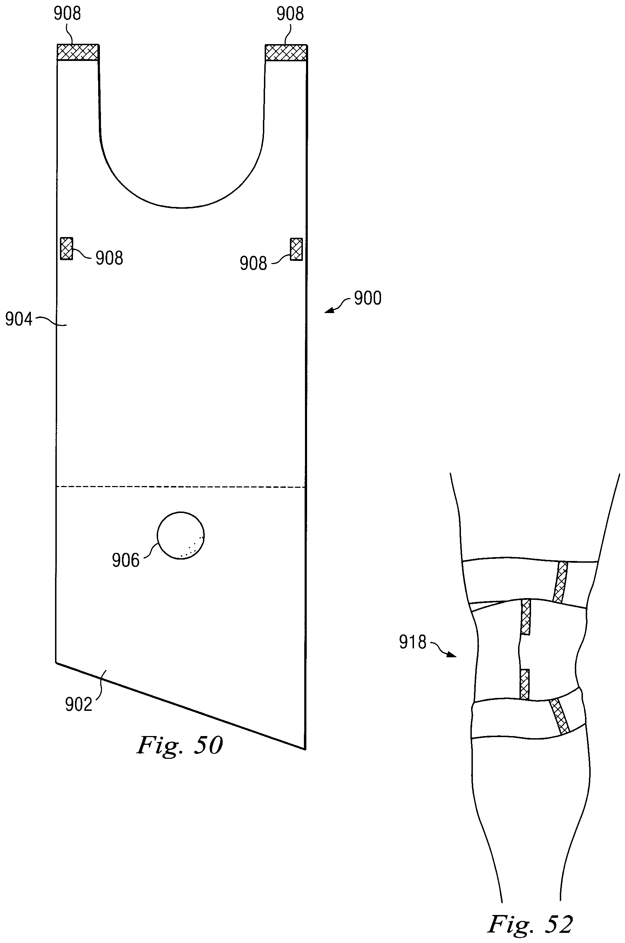

Fig. 50 shows another kneepiece referenced herein by the numeral 900. In this embodiment, the kneepiece 900 is formed of a first material 902 and a second material 904. These may be attached using methods known in the art. Here, the first material is a long stretch material and the second material is a different material, such as a short stretch material. In use, the circle 906 fits over the kneecap and the band is then wrapped around the leg. Because of the U-shape, when the band is wrapped around the leg, the U-shape portion straddles the kneecap and connectors 908 hold the kneepiece in place.

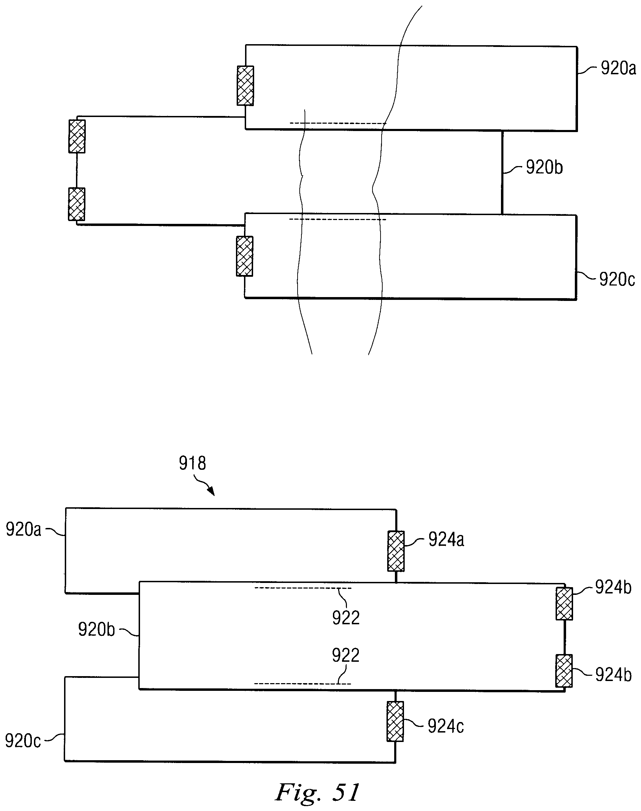

Fig. 51 is another kneepiece referenced by the numeral 918. In this embodiment, the kneepiece comprises three bands 920a-920c placed adjacent each other. The bands are connected along seam lines 922 extending substantially parallel to lateral edges of the bands. Connectors 924 are located at one or both ends of the bands and connect the band ends to the surface of the bands when wrapped around a limb.

Fig. 52 shows how the kneepiece 918 of Fig. 51 is applied to the knee joint.

The padding used in the joint pieces may be selected from various foams, and spacer fabrics, applied as one or a plurality of layers, and with profiles that include flat, channeled, grooved, with one or more holes, slotted, dimpled, raised, or any other profile as desired.

Trim-To-Fit Business Method

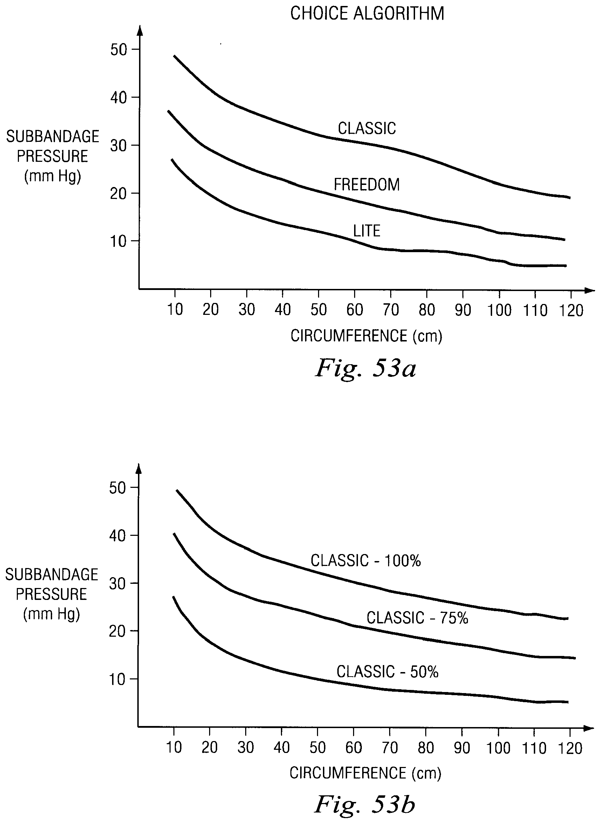

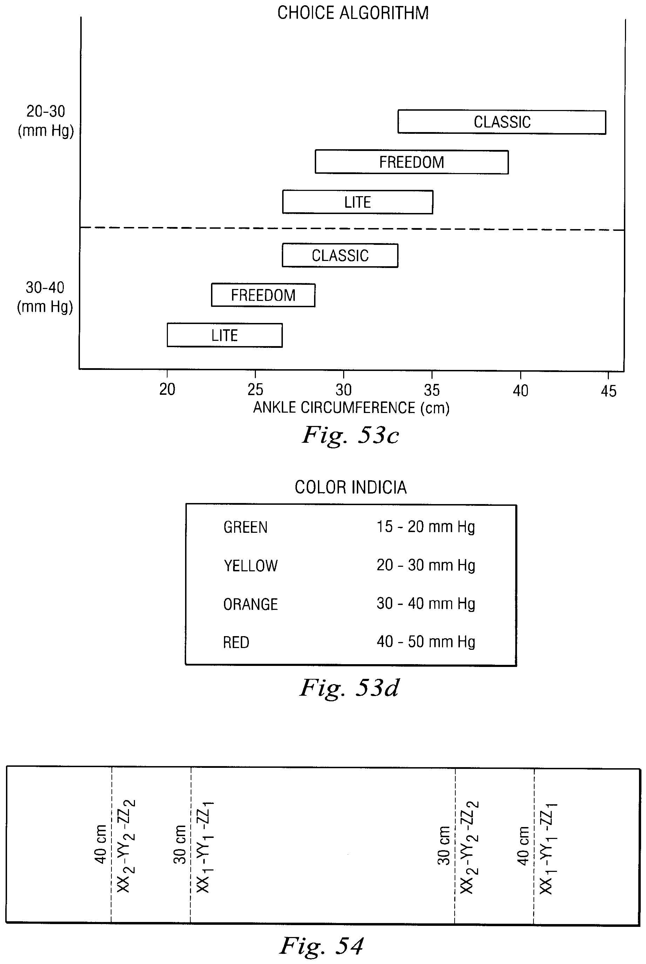

In another embodiment, a method and system are disclosed for providing custom and or customized garments to patients from off -the-shelf or trim to fit sizes / components based on limb measurements. This is explained further with reference to Figs. 53-58.

Laplace's law dictates the correlation of leg circumference with compression level. For smaller limbs, there is more compression on a limb portion than for a larger limb. The width of the bands also is an important factor when determining the compression level. For more narrow bands applied with the same tension, there will be more compression on the underlying limb portion than if the bands have wider widths. If the band is applied with the same tension to a limb portion with wider circumference, there is less tension per area on the underlying limb portion.

Therefore, to accurately gauge the compression applied to the limb at rest, one must take into account the limb circumferential measurements.

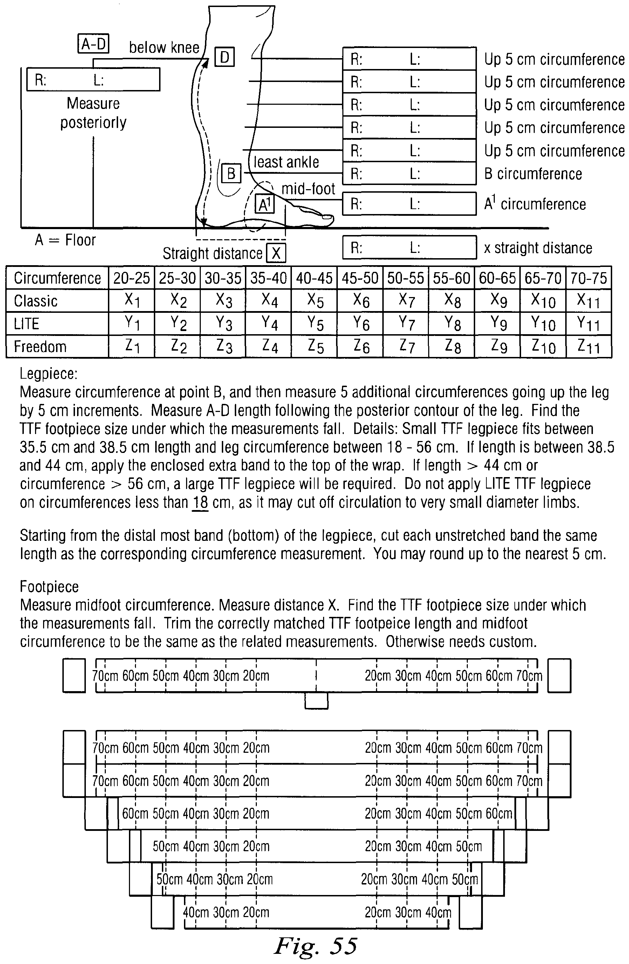

Fig. 55 shows an example of a form with instructions for custom fitting an off-the shelf garment. A user measures the actual circumference of an affected limb. Then, based on the type of material desired and the required compression level, a Choice Algorithm would help the user to select a correct product line. The Choice Algorithm graph, as shown in Fig. 53a indicates the proper resting compression of different band types (along the vertical axis) for a given limb circumference (along the horizontal axis) when bands are supplied at end stretch. The compression levels would be different, depending upon the type of material chosen allowing for choice when prescribing the resting compression level. The band type rating includes variations due to Laplace's law, which states that compression differs with limb circumference. In this example, these are short-stretch bands applied at or near end-stretch. In other embodiments, these may be other band types nonelastic, long-stretch, medium-stretch. One can use similar algorithms for maximal stretch (100% stretch), 75% maximal stretch, 50% maximal stretch for a given stretch material, for example, as illustrated in Fig. 53b. Other possibilities exist as well. Instead of displaying the subbandage pressure, the Choice Algorithm may display the Skin Surface Pressure and take into account the type of padding used under the wrap.

For example, a band applied over a thick foam liner with 1" of foam would provide less Skin Surface Pressure than a band applied over a very thin 2mm thick foam liner. Thus, the user can use a simple chart to correctly select which padding type and band type to use for a given application or band level.

Fig. 53C shows another embodiment of a Choice Algorithm. In the United States, most compression garments have a compression rating that lies in the ranges of: 8-15mm Hg, 15-20mm Hg, 20-30mm Hg, 30-40mm Hg, or 40-50mm Hg. Most practitioners are used to prescribing a range of therapeutic compression, with the understanding that of-the-shelf garments provide this compression range over a range of limb circumferences. The rating system typically has the most compression at the wrist or ankle, with less compression proximally so that graduated compression is provided. Short-stretch products as illustrated in this invention may or may not provide graduated compression, as this may in certain cases be less important for garments applied at or near end-stretch.

Fig. 53D illustrates a Color Indicia System for different ranges of compression. Since 15-20mm is generally safe except for severe peripheral arterial disease, it was given the color green. As the compression increases, the colors change to reflect more compression and therefore more caution. Red for instance, is often associated with stop lights, stop signs, or thermometer temperature level, as well as blood, and in this case represents higher intensity compression. The colormap shown in 53D may be used in any of the Choice Algorithms or bands to indicate general compression application. The colormap, for example, may be printed on the bands and reflect quickly the general compression level (safe to high intensity) either alone or in conjunction with other indicia. Other colormaps or ranges are possible, for instance using French or German Raul compression ranges, which are different than those commonly used in the United States. The colormap can be used in Fig. 55, for example. For the lower extremity garment pictured, additional color indicia may be added to show compression ranges. It is understood to one knowledgeable in the art that there may be different colormaps even on the same band, depending on the circumference measurement. Such color indicia may provide safety factor as well in helping the person trimming quickly double check compression ranges (make sure there are no red indicia if applying this to a patient with moderate / advanced peripheral arterial disease, for example).

For Fig. 53C, the color indicia may be used to provide color to the rectangles, which show the compression rating for certain circumferences. Since lower extremity compression garments are generally rated by the least ankle circumference compression rating, this chart shows the compression level ratings. Note that in this example, the same product can provide 20-30mm or 30-40mm compression, depending on the circumferences to which the product is applied. By mixing the band composition, band width, considering the circumference of the limb portion at that band level (in order to adjust for Laplace's law), as well as considering band overlap (in this case we mean how much each band overlaps on the preceding or immediately following band level. For example, creating a garment with 50% band overlap would give effectively two bands overlapping along the entire garment except at the ends and would give effectively twice the compression as if the bands did not overlap), many different combinations can be created to apply proper therapeutic compression levels, yet the details of all these many complicated variables remain hidden to the user, who gets a simple system to help them safely and reliably apply the correct compression level to the patient and correctly select the band materials or product and trim garment appropriately.

An indicia system can be drawn on, sewn in, or otherwise associated with the band, telling the user where to cut the band for a given circumference in order to get the correct band length for the desired compression. One example of this is to create a compressed scale. For example, a limb measuring 100cm in circumference may be treated with a 50% maximal stretch short-stretch band. When applied at end-stretch, the garment would apply, for example, 30-40mmHg range resting compression over a range of circumference limb portions. The band may contain an indicia system with simple numbers, telling the user to cut the band there. Since the band only needs to be 100cm / 150% long to be applied at end-stretch, the indicia system would tell the user to cut the band at a point slightly longer than the 66.67 measurement. Because of possible user error and prudence to leave room for the garment to work even with some leg swelling, the corresponding indicia for a 100cm limb at points would be put such that the user would cut the unstretched band off at 75cm in length. This point would be 75/2 out from the middle of the band, which is 37.5cm from the midpoint on either side. At this location on either side of the band, the indicia would say 100 or 100cm or something similar. There would be similar indicia for other limb measurements of 90cm, 110cm, etc. such that the user would know where to cut to make the garment the proper length to provide the desired compression to a limb.

The method includes taking measurements of limb sections, then using an algorithm which takes into account the stretch characteristics of the material, to figure out what length to cut the band. The algorithm takes into account the maximum stretch of the material and tells the user what unstretched length to cut the band. This measurement would be made outward from each of the spines or include a double ruler which has middle as the zero point and counts up on either side. Also, the bands could in some embodiments be cut at the actual circumference measurement of the limb for greatest intuitiveness.

Preferably a disposable or reusable ruler would be used which would tell the user where to cut each band. For different compression levels, there would be different algorithms. For example, one compression band may have maximum stretch of 50%. The compression when applied at end stretch to one ankle size may be 40mmHg, or some other predetermined compression amount. The ruler would tell the user where to cut the bands so that when they are overlapped, the band applies a compression level of, for example, 40mmHg at or near maximal stretch. In another instance, we may want to use the same garment to only provide 20mm compression. In this case, the compression at half maximal stretch would be 25% stretch and may correlate to 20mmHg. Another ruler or indicia would tell the user where to cut the band such that when overlapped on the limb the band applies a correct compression level of 20 mmHg.

To elaborate on this concept, we give the formula below which helps the manufacturer develop a compression ruler or indicia system which takes into account all the variables. This indicia system created may be printed on a disposable paper double ruler included with the product or a reusable ruler, or as indicia printed on the band, to name just a few methods of relaying such information. In one example, compressed length markings are used on bands that consider the amount of stretch and desire to leave some lateral overlap at each band level (ex. 20-50% overlap after garment applied correctly). The user would measure the actual limb circumference and cut along the markings / indicia on the band(s) that match the measured circumference. The user may use an optional measurement guide and instructions such as provided in Fig. 55 to assist with proper measurements at each band level. This could be a direct 1:1 relationship or some other relationship.

For a 1:1 relationship, the user would measure the limb circumference, and then cut each band the same length as the circumference of the limb measured for that limb level. For a better fit, however, a formula could be used to create a compressed length at which each unstretched band should be cut. This compressed ratio would take into account the degree of stretch at application of the garment, and perhaps include a fudge factor in case of diurnal variation in swelling, mis-measurements, etc. One example of this compressed ratio would be as follows: The limb measures 100cm in circumference at a level. The band is a short-stretch band with 50% maximal stretch. The length of the band once applied at maximal stretch would be as follows:

$$BLn = ((LMa+PL) / (1+ BSA)) * (1+ PBOd)$$

Where BLn - Band Length Needed for that limb level

LMa =actual Limb Measurement at that limb level

BSA = Percent band stretch at application

PBOd = Percent Band Overlap desired

PL = Padded Liner extra circumference at that limb level

For this application BLn = 100/1.5 = 66.67cm. The Percent Band Overlap desired is perhaps 20%, as we want to include extra length for possible measurement error and leave room for the garment to still work if patient's limb swells 20% over the circumference measurement at the time of fitting / trimming. In this case the patient will be using a sock type liner under the wrap, so the PL will be negligible and estimated at zero. If there were a padded liner used on the limb, the PL would add the additional circumference of the padded liner to that limb portion to the calculation. If the measurements of the limb LMa were done over a patient with the liner already applied, then PL would be zero for all calculations as the LMa would already include the extra circumference of the padded liner. In this case, the compressed scale would have actual length of 66.67*1.2 or 80cm. So for this compressed scale example, we would want the band at this band level cut to have 80cm total unstretched length for proper fit.

The compressed indicia system can be done several different ways in order to make it easier to trim and fit the garment for the user. One way is to print a compressed scale indicia on the band. Another method is to use a double zero ruler which the person fitting would use to trim the band appropriately. This may be the end user or a medical professional, a hospital personnel, or a person at a Durable Medical Equipment store. The compressed scale would tell the user to cut the band length 80cm (40cm on either side of the midpoint) by using some type of indicia. In this example, the printed number may simply read "100" or "100cm" at band points 40cm from either side of the band midpoint. By calculating and printing indicia at regular intervals (every 5-10cm for example using the compressed scale), the user would simply measure the limb at each band level and then cut at the location closest to their actual limb measurement. The built in scale will have done all the calculations in order to reduce risk of user error and make trimming as simple and uncomplicated as possible.

In other embodiments, the results of the compressed scale would be in a table such as shown in Fig. 55. In the case of the table of Fig. 55, for a band level circumference in the range of 20-25cm, the user would cut the band length at X1 for Classic band type. If the measurements was 25.1 - 30cm, the user would cut at X2. The table may reflect only one band composition such as a Classic brand band, or may include multiple types of product lines, such that the chart can be used for any product type. The table in Fig. 55, for example, shows where to cut for the Classic, Freedom, and Lite materials. This may be indicated if the different materials have different stretch levels.

In other embodiments, the chart illustrated in Fig. 55 may be different. For example, in some embodiments, it may tell the user exactly where to cut the bands to achieve 100% maximal stretch for the first line of the chart X1-X11, 75% maximal stretch for the second line of the chart Y1-Y11, and 50% maximal stretch for the third line of the chart Z1-Z11. Since the band would be trimmed to exactly correct length, the user would apply the garment with exact specified compression, even if not applied to end-stretch. For example, if we want a user with moderate peripheral arterial disease and diabetes with neuropathy to apply a Classic garment, we may use a chart with indicia to trim the garment at 50% maximal stretch, in order to provide 15-20mm compression at the ankle. This would provide a safe therapeutic compression level for this patient, even though garment is not applied at end-stretch. For a different patient who has no peripheral arterial disease but has severe lymphedema, we may use a chart to trim the garment to fit at 100% maximal stretch to provide 40mm end-stretch compression at the ankle and maximize the calf-muscle pump as well as prevent any additional swelling, since the band is applied at end-stretch.

Thus the chart provides another method of taking complicated variables and summarizing them in a simple and reliable method to make a one-time change to the garment which will permanently change the fit and performance characteristics of the garment, depending on patient's limb sizes and desired performance characteristics.

For another application, the chart may indicate where the user should trim the bands to provide a predetermined compression level (for example 30-40mm Hg range compression). In this case, the chart would take into account the degree stretch of each band type in order to get the proper level therapeutic compression.

For a padded liner application, the compressed scale in some embodiments would already consider the extra length needed for the band, depending on the application. For example, a padded liner as shown in Fig. 2 in one embodiment may increase circumferential measurement of a limb portion such as the Least Ankle Circumference by 4cm. Therefore for the following example PL = 4. In this example, we will use a short-stretch band with 34% maximal stretch and use the formula to properly size a band around a 27cm ankle. We will include just 5% overlap, so POd will be 0.05. In this case, the calculation becomes: BLn = ((27+4) / (1+0.34))*(1+0.05) = (31/1.34) *1.05 = 24.29cm. For a 73cm thigh measurement with PL of 6cm and desired overlap of 10%, the calculation would be BLn = ((73+6) / (1+0.34))(1+0.1) = (79/1.34)*1.1 = 64.85cm. Thus, by building in the desired padding, the desired band overlap, and knowing the percentage stretch of a short-stretch band, a system can be built so the user can easily and with low error correctly trim a garment to properly fit a limb portion.

Similar calculations can be used for any stretch material, such as the trimmable footpiece segments shown in Fig. 38. In this case, the indicia lines may be marked with the compressed scale in order to show the person trimming the garment how to trim it to properly fit the limb. Other indicia line types are possible for footpiece segments, and indicia may reflect something simpler for the user such as trimming along indicia which vary depending on shoe size or other type markings (small, medium, large, for example).

For different types of materials, there may be different amounts of stretch. Choice algorithm Fig. 53A shows a representative graph of different pressures of short-stretch bands applied to different circumference bands. All points are for bands applied at end-stretch. The user could look at the circumference of the limb portion and the desired compression level the healthcare practitioner wants to apply, and select the proper band type to use. In Fig. 53A, for instance, a 40cm circumference ankle would have about 15mmHg resting compression for the LITE, 325mm for the Freedom, and 35mm for the Classic when applied at or near end stretch. In one example, the patient has diabetes with moderate peripheral neuropathy and some Peripheral Arterial Disease, but still needs lower extremity compression. In consideration of the patient's comorbidities the health care practitioner may select to prescribe the Lite material for the patient. In another example, the patient has good blood flow and severe lymphedema with ankle measurement of 40cm. In this case, the health care practitioner would want heavier compression and would select the Classic, with 35mm Hg resting compression on the ankle area.

For selection of garments for use over the padded liner, the skin surface pressure on the limb will be different. The skin surface pressure we will define as the pressure on the outermost layer of skin. Therefore, the Choice algorithm would look different depending on the liner material and thickness and construction. These figures could in some embodiments give the correct Skin Surface Pressure, rather than the SubBandage Pressure. In this case, the Choice Algorithm would have lower mmHg reading built into the scale (giving correct skin surface pressures rather than subbandage pressures), to aid the practitioner with proper selection of band materials in order to provide proper therapeutic compression. It is important to note that the skin surface pressures measured also depend on tissue softness. This means a posterior calf skin surface area would be lower due to softer underlying tissues than a reading over the anterior tibial crest, which is quite bony. All these considerations can be built into the correct scales to make a solution that is as simple and seamless as possible for clinician, technician, and patient.

Alternatively, the indicia showing where to cut may additionally or alternatively indicate the resting compression level applied to a specified circumference when applied at end stretch. Fig. 54, for example, shows bands with up to three compression ratings XX-YY-ZZ where XX is resting compression level at maximal band stretch, YY is resting compression level at 75% maximal stretch, and ZZ is the resting compression level at 50% maximal stretch. These ranges were chosen in particular because users can determine not only the maximal end-stretch of short-stretch materials, but some general range of maximal stretch as well with reasonable predictability and reliability. By selecting the correct size and trimming correctly, the proper compression level for a given circumference can be determined by the user. These indicia may contain additional colormaps or use colormap blocks instead of numbers in order to indicate ranges of compression. One benefit of such a system is that this needs to be determined only once at time of garment selection and trimming, then the garment can be given to the patient and the patient can reliably and predictably apply the garment with correct and safe compression levels and have a properly fit garment. In this example the band cut along the 30cm line (which could represent actual measurement or a compressed measurement as described by equations above), the user would get resting compression of XX1 mmHg compression if the band was applied at maximal stretch. If applied at 75% maximal stretch, for a 30cm band the user would get a resting compression of YY1 mmHg. If the user applied the garment at 50% maximal stretch for a 30cm band, the user would get a resting compression of ZZ1 mmHg. Thus, the current invention proposes a correct rating system of compression to determine actual subbandage pressure or skin surface pressure of a garment. The compression rating system could similarly include different ratings depending on the padding selected or other criteria.

Such a system has clinical benefits. For example, as lymphedema patient wears a Classic garment that provides 40mm resting compression to the ankle at a given diameter 35cm in circumference. The patient is to undergo surgery elsewhere on the body. Because surgery often involves fluid fluctuations due to IV fluids, blood loss, blood pressure fluctuations due to analgesia, etc, some patients get significant worsening of their lymphedema. At the same time, it is the author's experience that greater than 20mmHg for a surgical patient may be dangerous because of anesthesia causing lower blood pressure and perfusion pressure, analgesias on board etc. At the same time, lower extremity compression is necessary and useful to lower incidence of deep venous thrombosis. Therefore, by utilizing the rating system, the correct compression for maximal therapeutic purposes and maximal safety may be 20mmHg end-stretch. In this example, the patient may look at their garment and note that at 35cm their compression rating is 40-30-20. In this case, the patient understands they can apply their garment at 50% maximal stretch and get 20mm compression to their limb, even thought it is not applied at end-stretch. In this case, their garment with 20mm compression would certainly be safer than wearing no garment at all (which would increase risk of blood stasis and DVT formation), and safer than wearing a 40mm garment during surgery.

In other embodiments, the indicia may be linked to the amount of internal compression applied to the tissues inside the limb portion, which is different from the surface compression applied by the garment either with or without a padded liner. Thus both the practitioner and/or end user would know both the external compression generated as well as the internal compression to the limb as both could be printed on the indicia.

It is important to consider hand strength necessary to pull bands to appropriate tension. Most doors are rated to open with pulling strength less than 5 lbf of force. It is desirable, therefore, that the bands be configured such that the tension needed to pull the bands to near or at end-stretch be lie inside of a range of 1 to 10 lbf. In some preferred embodiments, the desired tension lies within a more narrow range of 3-5 lbf. The amount of tension to achieve the compression could be varied by narrowing the bands or providing more overlap on the bands in order to keep required hand tension within an acceptable range, while providing a garment with the correct amount of therapeutic compression to the underlying limb. Lower required pull forces are preferable so that patients with less hand strength can still apply the garment correctly and with reliable and predictable compression levels.

For far more elastic (beyond 100% stretchability) bands, geographic markings (rectangle-square or oval-circle) may be used with appropriate stretch and then trim-to-fit markings. Squares may be placed on the device at the proper stretch ratio that would generate the requisite compression on the circumference that the garment is trimmed for. For short-stretch bands, these indicia may provide additional confirmation the garment was applied correctly for best fit and function.

Any embodiment presented above with ruler, chart, or print-out on the band may be used. Also, a built in 20-50% compressed ruler may be used for the customer to cut bands such that there would be a fudge factor built in to allow for some user error, swelling fluctuation, etc.

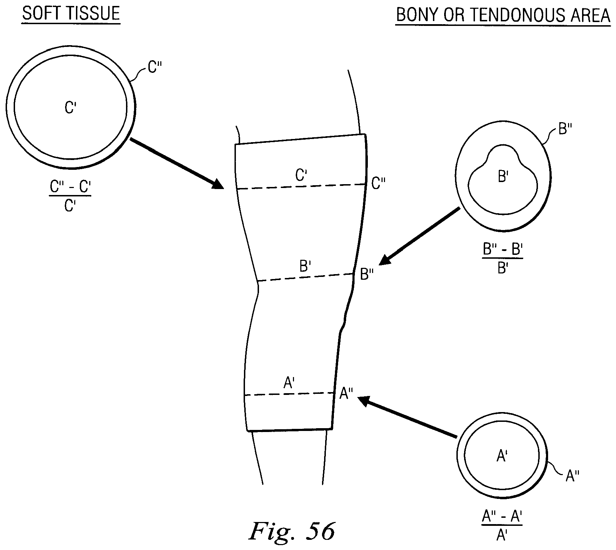

Fig. 56 shows a relationship for at least partially determining a pressure applied to a limb at a given cross-section based upon the cross sectional area of the liner. This may be used for either a compression liner to be used beneath a garment or the garment itself. Fig. 56 shows a leg, about which a liner is applied. A cross section of the liner is shown at three locations along the limb at A, B, and C. Each cross-section of the liner has an outer circumference and an inner circumference. The inner circumference is equal to the circumference of the limb.

The compression at each cross-sectional location is based upon the difference in circumference, divided by the inner circumference. Using this relationship, compression can be calculated even when the thickness of the padding used in the garment differs from cross-section to cross-section.

Determining a pressure exerted on the limb at the cross-section may be accomplished using a first function of the ratio described above (that is, taking the difference in circumference, divided by the inner circumference) and using a second function of the inside circumference at the same cross-section. For explanation, the following equations are provided and can be understood with reference to Fig 56. For equal padding:

$$\frac{C'' - C'}{C'} < \frac{B'' - B'}{B'}$$

and

$$\frac{A'' - A'}{A'} < \frac{B'' - B'}{B'}.$$

However, if less padding necessary at the areas having more bony tissue, then depending on the amount of padding:

$$\frac{A'' - A'}{A'} > \frac{B'' - B'}{B'}$$

and

$$\frac{C'' - C'}{C'} > \frac{B'' - B'}{B'}.$$

$$\frac{A'' - A'}{A'} < \frac{B'' - B'}{B'}.$$

In simple terms, using a Laplace equation that shows a pressure **P** is proportional to the applied tension **T** and is inversely proportional to the circumference **C**, and with a constant **K** of proportionality, the following can be written:

$$P = KT/C$$

If two circumferences are given, an outer circumference **C1** and an inner circumference **C2**, with **C1** being the larger circumference, the difference in pressure at **C1** as compared at **C2** can be expressed as:

$$P1 - P2 = KT/C1 - KT/C2$$

Since pressure P1 is at the larger outer circumference, **C1**, It will be indicated by **Ppad**, for pressure at the padding. Likewise, pressure **P2** is at the smaller inner, or limb circumference, **C2**, so It will be called **Plimb.**

Thus:

$$Ppad - Plimb = KT/C1 - KT/C2$$

Now if only circumferences **C1** and **C2** are taken into account, they can be related to the difference in pressure **Ppad** - **Plimb** with the normalized factor,

$$(C1 - C2)/C2$$

as follows:

repeating equation 3)

$$Ppad - Plimb = KT/C1 - KT/C2$$

and using the normalized factor **(C1** - **C2)/C2** with **Ppad,** or **KT/C1,** it can be written:

$$Ppad - Plimb = \{ KT/C1 \} \times \{ (C1 - C2)/C2 \}$$

By multiplying the numerators and the denominators at the right of the equality, it can be written:

$$Ppad - Plimb = \{ KT(C1-C2)\} / \{C1C2\}$$

Algebraic manipulation gives:

$$Ppad - Plimb = KTC1/C1C2 - KTC2/C1C2$$

And then:

$$Ppad - Plimb = KT/C2 - KT/C1$$

Note that this derivation reproduced equation 3) with one striking difference, the sign on the right hand of the equality is reversed. To correct the right hand side of the equality it can be written:

$$(Ppad - Plimb) \times (-1) = KT/C1 - KT/C2$$

The minus 1 indicates a pressure drop when going from the smaller circumference to the larger circumference.

It is important to note that the pressure at **C1,** or **Ppad,** is the pressure exerted on the padding, not on the limb. The limb experiences a pressure that is even lower than **Ppad.** The simplest example to illustrate this is with a fluid padding (air or liquid).

According to the laws of hydrostatics, the pressure in the fluid padding must be equal at all points, otherwise it would not be static. Since the pressure is everywhere equal, the force per unit area is also everywhere equal. However, the area at the limb surface, at **C2,** under the padding is smaller than the area at **C1** the subbandage area. Thus, the smaller area at the limb has less total force exerted on it than at the larger area at **C1.** A reverse analog to this (the principles being exactly the same) is a hydraulic braking system in a car. A small force exerted by the foot on a small diameter piston results in a large force exerted on a relatively larger piston which presses the brake discs to the rotor.

Business Method for Padded Liner

By using a form, the end user or business or clinical person can fill out the form to select the correct area(s) to be padded and the correct height of padding. The user can further select the correct thickness or layers of padding. This form may then be submitted to a manufacturer for custom manufacturing of the desired liner.

In some aspects, the present disclosure is directed to a liner for decreasing subbandage pressure to bony areas of a patient's limb during treatment with a compression garment while maintaining a lower profile in less bony areas, the liner comprising a tubular body portion having a proximal opening for receiving a limb and being sized to extend about the circumference of the limb, the body portion including a first region having relatively less padding and a second region having relatively more padding, the second region being located on the tubular body portion to align with relatively harder tissue areas of the limb, the first region being located on the tubular body to align at least in part with relatively softer tissue areas of the limb, wherein the second region comprises a padding material formed of spacer fabric, the spacer fabric having first and second substantially parallel layers separated by threads that provide compression resistance and that permit air between the layers and breathability of the limb.

In different exemplary aspects: The padding may beveled at edges providing a transition in thickness from the second region to the first region. The body portion may comprise a pocket and the second region may be formed of a selectively removable pad insertable into the pocket. The second region may comprise a plurality of regions having different padding thicknesses. The tibia includes 0.2cm padding and the anterior ankle portion includes 0.8cm padding. The padding in the second region is about .1-.2cm thick. The second region comprises a padded material permanently fixed to the tubular body portion. The liner has a length sufficient to extend from a patient's foot to a patient's thigh. The second region covers the first and fifth metatarsals. The second region is formed of a laminate of a plurality of padding pieces. The second region comprises a narrow spacing between adjacent padding pieces, the narrow spacing being disposed on the liner to overlie the anterior tibial crest and to distribute compression loading to the either side. The second region covers the anterior ankle and comprises a plurality of slits or grooves extending laterally across a central portion of the second region. The second region comprises a plurality of channels formed as indentations in the second region. The second region is arranged in the body portion to cover an anterior ankle region, a malleoli region, and a tibia region of the limb.

In some aspects, the present disclosure is directed to a low profile liner for providing increased comfort to a patient's limb during treatment with a compression garment by distributing subbandage pressure at bony areas of the limb. The liner comprises a tubular body portion having a proximal opening for receiving a limb and being sized to extend about the circumference of the limb, the body portion including a first region having relatively less padding and a second

23

region having relatively more padding, the second region being located on the tubular body portion to align with relatively harder tissue areas of the limb, the first region being located on the tubular body to align with relatively softer tissue areas of the limb, the tubular body portion having a beveled edge portion transitioning from the second region to the first region.

In some aspects, the present disclosure is directed to a system for applying therapeutic pressure to a patient's limb. The system comprises: a liner comprising a tubular body portion having a proximal opening for receiving a limb and being sized to extend about the circumference of the limb, the body portion including a first region having relatively less padding and a second region having relatively more padding, the second region being located on the tubular body portion to align with relatively harder tissue areas of the limb, the first region being located on the tubular body to align with relatively softer tissue areas of the limb. The system also comprises a compression garment having a plurality of stretchable bands wrappable about an exterior of the liner, the stretchable bands being adjustable to provide a therapeutic level of compression to the limb, the compression liner being adjacent to and providing compression to both the first and second regions of the liner.

In some aspects, the present disclosure is directed to a method of treating a condition of a patient's limb. It comprises: applying a liner to the limb, the liner comprising a tubular body portion having a proximal opening for receiving a limb and being sized to extend about the circumference of the limb, the body portion including a first region having relatively less padding and a second region having relatively more padding, the second region being located on the tubular body portion to align with relatively harder tissue areas of the limb, the first region being located on the tubular body to align with relatively softer tissue areas of the limb. The method also comprises aligning the second region of the liner to overlie the relatively harder tissue on the limb and applying a compression garment about the liner to apply a therapeutic range of compression to the limb.

The present disclosure is directed to a therapeutic compression apparatus comprising a liner having a tubular body portion with a proximal opening for receiving a limb of a patient; said tubular body portion having an inside region with an inside circumference that is sized to extend about and in contact with the limb, the inside circumference being substantially identical to a circumference of the limb at a respective area of contact; the tubular body portion having an outside region, the outside region having an outside circumference that varies from the inside circumference at respective cross-sections through the tubular body, such that a first function of a ratio of the difference between the inside circumference and the outside circumference at a respective cross-section through the tubular body, divided by the inside circumference at said respective cross-section, and a second function of the inside circumference at said respective cross-section determines, in part, a pressure exerted on the limb at said cross-section when compression is applied by said therapeutic compression apparatus.

In one aspect, the tubular body portion of the liner further comprises an outside cross-sectional area circumscribed by said outside circumference; an inside cross-sectional area circumscribed by said inside circumference; a padding area being the difference between said outside cross-sectional area and said inside cross-sectional area; a first region having a first integrated plurality of padding areas; a second region having a second integrated plurality of padding areas; the first and second region each having padding characterized by dimensions, properties and profiles; the padding in the first region has a dimension that is relatively thicker than the padding in the second region, and the padding in the second region has a dimension that relatively thinner than the padding in the first region; the first region being located on the tubular body portion to align over relatively bony and tendinous areas of the limb; and the second region being located on the tubular body portion to align at least in part with relatively softer tissue areas of the limb.

In one aspect, the padding is taken from the group consisting essentially of spacer fabrics, open-cell foams, closed-cell foams, viscoelastic foams, fluid filled elastic enclosed paddings, silicons, terry cloths, wadded fabrics, flexible aerogel blankets, gases, injected single or multipart foams, downs, metal and non-metal springs, inorganic polymers, Newtonian and non-Newtonian fluids, dilatant fluids, synthetic and natural rubbers, and magnetic repulsion paddings.

In one aspect, the liner further comprises at least one opening in the first region for accepting insertable and replaceable padding.

In one aspect, the liner further comprises at least one attachment mechanism for replaceably attaching padding.

In one aspect, the liner further comprises at least one attachment for permanently fixing padding.

In one aspect, the liner further comprises padding having a thickness that transitions from a thicker padding in the first region to a thinner padding in the second region.

In one aspect, the liner further comprises padding having a transition from a padding in the first region to no padding in the second region.

In one aspect, the liner further comprises padding having a thickness ranging from 0.1 to 2.0 centimeters thick.

In one aspect, the liner further comprises a first region that covers the first and fifth metatarsals.

In one aspect, the liner further comprises a first region that is formed of a laminate of a plurality of padding pieces.

In one aspect, the liner further comprises a first region having narrow spacing between adjacent padding pieces, the narrow spacing being disposed on the liner to overlie the anterior tibial crest and to distribute compression loading to either side.

In one aspect, the liner further comprises a first region that covers the anterior ankle and comprises a plurality of slits, grooves, and holes extending laterally across a central portion of the first region.

In one aspect, the liner further comprises a first region with a plurality of channels formed as indentations in the first region.

In one aspect, the liner further comprises a first region arranged in the tubular body portion to cover an anterior ankle region, a malleoli region, and a tibia region of the limb.

In one aspect, further comprising padding that includes channeled spacer fabric.

In one aspect, further comprising padding that includes waffled and grooved spacer fabric.

In one aspect, further comprising foam padding having chipped, chunked and channeled profiles in the same apparatus.

In one aspect, further comprising padding having profiles that include slots, holes, channels, linear grooves, non-linear grooves, cross-hatched grooves, dimples, arrays of dimples, raised areas, arrays of raised areas, and combinations of profiles.

In one aspect, further comprising a plurality of bands wrappable about an exterior of the liner and attachable to each other, the bands being adjustable to provide a therapeutic level of compression to the limb, the therapeutic compression apparatus being adjacent to and providing compression to both the first and second regions of the liner.

In one aspect, further comprising a plurality of markings on said plurality of bands that indicate proper locations for trimming the bands to accommodate different limb sizes, said proper locations being determined by the first function, the second function and other parameters to provide proper therapeutic levels of compression to the limb.

In one aspect, further comprising bands made from spacer fabric that provide 20mm Hg compression to the limb when the bands are applied at end-stretch.

In one aspect, providing therapeutic compression to the limb further comprising one or more electrical conductive pads in contact with the limb, the conductive pads transmitting electrical pulses from a controller to motor nerves that stimulate contraction of muscles in the compressed limb, the therapeutic compression apparatus thereby providing increased pumping of venous blood through the limb.

In one aspect, the liner further comprises a length sufficient to extend from a patient's foot to a patient's thigh.

The present disclosure is directed to a method for applying therapeutic compression to a limb comprising inserting the limb into a liner having a tubular body portion with a proximal opening for receiving the limb of a patient; said tubular body portion having an inside region with an inside circumference that is sized to extend about and in contact with the limb, the inside circumference being substantially identical to a circumference of the limb at a respective area of contact; the tubular body portion having an outside region, the outside region having an outside circumference that varies from the inside circumference at respective cross-sections through the tubular body; calculating a first function using a ratio of the difference between the inside circumference and the outside circumference at a respective cross-section through the tubular body, divided by the inside circumference at said respective cross-section; calculating a second function using the inside circumference at said respective cross-section; and determining, in part, a pressure exerted on the limb at said cross-section when compression is applied by a therapeutic compression garment.

In one aspect, the method further comprising determining an outside cross-sectional area circumscribed by said outside circumference; determining an inside cross-sectional area circumscribed by said inside circumference; determining a padding area, said padding area being the difference between said outside cross-sectional area and said inside cross-sectional area; determining a first region having a first integrated plurality of padding areas; determining a second region having a second integrated plurality of padding areas; the first and second regions each having padding characterized by dimensions, properties and profiles; the padding in the first region has a dimension that is relatively thicker than the padding in the second region, and the padding in the second region has a dimension that is relatively thinner than the padding in the first region; locating the first region on the tubular body portion to align over relatively bony and tendinous areas of the limb; and locating the second region on the tubular body portion to align at least in part with relatively softer tissue areas of the limb.

In one aspect, the method of the padding is taken from the group consisting essentially of spacer fabrics, open-cell foams, closed-cell foams, viscoelastic foams, fluid filled elastic enclosed paddings, silicons, terry cloths, wadded fabrics, flexible aerogel blankets, gases, injected single or multipart foams, downs, metal and non-metal springs, inorganic polymers, Newtonian and non-Newtonian fluids, dilatant fluids, synthetic and natural rubbers, and magnetic repulsion paddings.

In one aspect, the method of further comprising inserting and replacing padding in at least one opening in the first region of the liner.

In one aspect, the method of further comprising attaching padding to the liner with at least one attachment mechanism for replaceably attaching padding.

In one aspect, the method of further comprising permanently fixing padding to the liner with at least one attachment.

In one aspect, the method of further comprising transitioning the padding from a thicker padding in the first region to a thinner padding in the second region of the liner.

In one aspect, the method of further comprising transitioning the padding from a padding in the first region to no padding in the second region of the liner.

In one aspect, the method of further comprising using padding having a thickness ranging from 0.1 to 2.0 centimeters thick on the liner.

In one aspect, the method of further comprising covering the first and fifth metatarsals with the first region of the liner.

In one aspect, the method of further comprising forming a laminate of a plurality of padding pieces for the first region of the liner.

In one aspect, the method of further comprising overlying the anterior tibial crest with padding having narrow spacing between adjacent padding pieces disposed on the liner at the first region, to distribute compression loading to either side.

In one aspect, the method of further comprising covering the anterior ankle with a first region of the liner that comprises a plurality of slits, grooves, and holes extending laterally across a central portion of the first region.

In one aspect, the method of further comprising forming a plurality of channels as indentations in the first region of the liner.

In one aspect, the method of further comprising covering an anterior ankle region, a malleoli region, and a tibia region of the limb with a first region arranged in the tubular body portion the liner.

In one aspect, the method of further comprising using padding that includes channeled spacer fabric.

In one aspect, the method of further comprising using padding that includes waffled and grooved spacer fabric.

In one aspect, the method of further comprising using foam padding having chipped, chunked and channeled profiles in the same garment.

In one aspect, the method of further comprising using padding having profiles that include slots, holes, channels, linear grooves, non-linear grooves, cross-hatched grooves, dimples, arrays of dimples, raised areas, arrays of raised areas, and combinations of profiles.

In one aspect, the method of further comprising using a plurality of bands wrappable about an exterior of the liner and attachable to each other, the bands being adjustable to provide a therapeutic level of compression to the limb, the therapeutic compression apparatus being adjacent to and providing compression to both the first and second regions of the liner.

In one aspect, the apparatus of further comprising generating a plurality of markings on said plurality of bands that indicate proper locations for trimming the bands to accommodate different limb sizes, said proper locations being determined by the first function, the second function and other parameters to provide proper therapeutic levels of compression to the limb.

In one aspect, the method of further comprising using bands made from spacer fabric that provide 20mm Hg compression to the limb when the bands are applied at end-stretch.

In one aspect, the method of further providing therapeutic compression to the limb using one or more electrical conductive pads in contact with the limb, the conductive pads transmitting electrical pulses from a controller to motor nerves to stimulate contraction of muscles in the compressed limb, the method providing increased pumping of venous blood through the limb.

In one aspect, the method of further comprising extending the length of the liner to a length sufficient to extend from a patient's foot to a patient's thigh.

The present disclosure is directed to a system for providing therapeutic compression comprising a liner having a tubular body portion with a proximal opening for receiving a limb of a patient; said tubular body portion having an inside region with an inside circumference that is sized to extend about and in contact with the limb, the inside circumference being substantially identical to a circumference of the limb at a respective area of contact; the tubular body portion having an outside region, the outside region having an outside circumference that varies from the inside circumference at respective cross-sections through the tubular body, such that a first function of a ratio of the difference between the inside circumference and the outside circumference at a respective cross-section through the tubular body, divided by the inside circumference at said respective cross-section, and a second function of the inside circumference at said respective cross-section determines, in part, a pressure exerted on the limb at said cross-section when compression is applied by said therapeutic compression apparatus.

In one aspect, the system of the tubular body portion of the liner further comprises an outside cross-sectional area circumscribed by said outside circumference; an inside cross-sectional area circumscribed by said inside circumference; a padding area being the difference between said outside cross-sectional area and said inside cross-sectional area; a first region having a first integrated plurality of padding areas; a second region having a second integrated plurality of padding areas; the first and second region each having padding characterized by dimensions, properties and profiles; the padding in the first region has a dimension that is relatively thicker than the padding in the second region, and the padding in the second region has a dimension that relatively thinner than the padding in the first region; the first region being located on the tubular body portion to align over relatively bony and tendinous areas of the limb; and the second region being located on the tubular body portion to align at least in part with relatively softer tissue areas of the limb.

In one aspect, the system of further comprising a plurality of bands wrappable about an exterior of the liner and attachable to each other, the bands being adjustable to provide a therapeutic level of compression to the limb, the therapeutic compression apparatus being adjacent to and providing compression to both the first and second regions of the liner.

In one aspect, the system of further comprising a plurality of markings on said plurality of bands that indicate proper locations for trimming the bands to accommodate different limb sizes, said proper locations being determined by the first function, the second function and other parameters to provide proper therapeutic levels of compression to the limb.

In one aspect, the system of further comprising bands made from spacer fabric that provide 20mm Hg compression to the limb when the bands are applied at end-stretch.

In one aspect, the system of providing therapeutic compression to the limb further comprising one or more electrical conductive pads in contact with the limb, the conductive pads transmitting electrical pulses from a controller to motor nerves that stimulate contraction of muscles in the compressed limb, the therapeutic compression apparatus thereby providing increased pumping of venous blood through the limb.

In one aspect, the system of the liner further comprises a length sufficient to extend from a patient's foot to a patient's thigh.

In one aspect, the system of further comprising at least one roll of liner material having a tubular body portions; the tubular body portions being dispensed in lengths from the roll of liner material; and the dispensed lengths of tubular body portions having padding attached to the first regions.

In one aspect, the system of further comprising padding extending linearly from a start to an end of the roll, such that dispensed lengths of the tubular body portions include padding from a start to an end of the dispensed lengths, the padding extending only partway around the circumference of the tubular body portions of the dispensed lengths.

In one aspect, the system of further comprising padding intermittently placed along the roll, such that each dispensed length of the tubular body portion includes padding in the first region that aligns over relatively bony and tendinous areas of a limb.

**Claims**

1. A trim-to-fit compression garment apparatus for treating a condition of a patient's limb, the apparatus comprising:
   material having properties of short stretch, with maximum elasticity of 15-100% such that the garment provides a compression level that falls within the range of 8-50 mmHg when applied on a resting supine limb at or near end-stretch,
   the material forming a plurality of bands wrappable about the limb, and attachment mechanisms to connect the ends of the plurality of bands;
   markings showing where to trim at least one band of the plurality of bands in order to fit a smaller size limb;
   **characterized in that**
   the marking comprising an indicia system drawn on, sewn in, or otherwise associated with the at least one band and telling a user where to cut the at least one band for a given circumference in order to get the correct band length for a desired compression; wherein
   the markings provide a compressed scale.

2. The apparatus of claim 1, wherein the markings are woven into fabric of the garment.

3. The apparatus of claim 1, wherein the markings are applied to the garment in an ink or dye.

4. The apparatus of claim 1, wherein the markings are guide patterns that overlay the garment and identify trim lines.

5. The apparatus of claim 1, wherein the garment further comprises:

   an upper band;
   lower bands; and
   an attachment mechanism attaching the upper band to the lower bands.

6. The apparatus of claim 1, wherein the garment comprises a footpiece.

7. The apparatus of claim 1, wherein the garment comprises a legpiece.

**8.** The apparatus of claim 1, wherein the markings are color coded.


**Patentansprüche**

**1.** Zuschneidbare Kompressionskleidungsvorrichtung zur Behandlung einer Beschwerde eines Körperglieds eines Patienten, wobei die Vorrichtung Folgendes aufweist:

Material mit Kurzzugeigenschaften mit maximaler Elastizität von 15 bis 100 %, so dass das Kleidungstück ein Druckniveau bereitstellt, das in einen Bereich von 8 - 50 mmHg fällt, wenn bei oder fast bei End-Dehnbarkeit auf ein ruhendes Körperglied in Rückenlage angewendet,
wobei das Material mehrere um das Körperglied wickelbare Bänder bildet, und
Anbringungsmechanismen zum Verbinden der Enden der mehreren Bänder;
Markieren, die zeigen, wo wenigstens ein Band der mehreren Bänder zuzuschneiden ist, so dass es einem kleineren Körperglied passt; **dadurch gekennzeichnet, dass**
die Markierung ein System mit Zeichen aufweist, die auf bzw. in das wenigstens eine Band aufgezeichnet oder eingenäht oder diesem anderweitig zugeordnet sind und einem Benutzer mitteilen, wo das wenigstens eine Band für einen jeweiligen Umfang zu schneiden ist, um die richtige Bandlänge für eine gewünschte Kompression zu erhalten; wobei
die Markierungen eine Kompressionsskala bereitstellen.

**2.** Vorrichtung nach Anspruch 1, wobei die Markierungen in Gewebe des Kleidungsstücks eingewebt sind.

**3.** Vorrichtung nach Anspruch 1, wobei die Markierungen in einer Druckfarbe oder einem Farbstoff auf das Kleidungsstück aufgetragen sind.

**4.** Vorrichtung nach Anspruch 1, wobei die Markierungen Anleitungsmuster sind, die das Kleidungsstück überlagern und Schnittlinien bezeichnen.

**5.** Vorrichtung nach Anspruch 1, wobei das Kleidungsstück ferner Folgendes aufweist:

ein oberes Band,
untere Bänder und
einen Anbringungsmechanismus, der das obere Band an den unteren Bändern anbringt.

**6.** Vorrichtung nach Anspruch 1, wobei das Kleidungsstück ein Fußstück umfasst.

**7.** Vorrichtung nach Anspruch 1, wobei das Kleidungsstück ein Beinstück umfasst.

**8.** Vorrichtung nach Anspruch 1, wobei die Markierungen farblich kodiert sind.


**Revendications**

**1.** Appareil de vêtement de compression ajustable pour traiter une affection d'un membre du patient, l'appareil comprenant :

une matière ayant des propriétés d'étirage court, avec une élasticité maximum de 15 à 100 % de telle sorte que le vêtement fournisse un niveau de compression qui se situe au sein de la gamme de 8 à 50 mm Hg lorsqu'il est appliqué sur un membre en décubitus au repos à l'étirement terminal ou proche de ce dernier,
la matière formant une pluralité de bandes aptes à être enroulées autour du membre, et
des mécanismes d'attache pour raccorder les extrémités de la pluralité de bandes ;
des marquages montrant l'endroit où il convient d'ajuster au moins une bande de la pluralité de bandes afin de l'adapter à un membre de taille plus petite ; **caractérisé en ce que**
le marquage comprend un système de repères dessinés sur, cousus dans, ou autrement associés à l'au moins une bande et indiquant à un utilisateur l'endroit où il convient de couper l'au moins une bande pour une circonférence donnée afin d'obtenir la longueur de bande correcte pour une compression désirée ; dans lequel
les marquages fournissent une échelle d'état compressé.

**2.** Appareil de la revendication 1, dans lequel les marquages sont tissés dans le tissu du vêtement.

**3.** Appareil de la revendication 1, dans lequel les marquages sont appliqués au vêtement avec une encre ou une teinture.

**4.** Appareil de la revendication 1, dans lequel les marquages sont des modèles-guides qui recouvrent le vêtement et identifient des lignes d'ajustage.

**5.** Appareil de la revendication 1, dans lequel le vêtement comprend en outre :

une bande supérieure ;
des bandes inférieures ; et
un mécanisme d'attache qui attache la bande supérieure aux bandes inférieures.

**6.** Appareil de la revendication 1, dans lequel le vêtement comprend une pièce pour le pied.

**7.** Appareil de la revendication 1, dans lequel le vêtement comprend une pièce pour la jambe.

**8.** Appareil de la revendication 1, dans lequel les marquages sont codés par couleurs.

*Fig. 1*

*Fig. 2*

*Fig. 3*

*Fig. 4*

*Fig. 5*

*Fig. 6*

*Fig. 7*

*Fig. 8*

*Fig. 9*

*Fig. 10*

*Fig. 11*

*Fig. 12*

Fig. 13

Fig. 14

Fig. 15

Fig. 16

*Fig. 17*

*Fig. 18*

*Fig. 19*

*Fig. 20*

*Fig. 21*

*Fig. 22*     *Fig. 23*     *Fig. 24*

*Fig. 25*     *Fig. 26*     *Fig. 27*

502 — 104

*Fig. 28*

502 — 104

*Fig. 29*

**Fig. 30**

**Fig. 31**

**Fig. 32**

**Fig. 33**

**Fig. 35**

**Fig. 34**

*Fig. 36*

*Fig. 37*

Fig. 38

Fig. 39

Fig. 40

*Fig. 41*

Fig. 42

Fig. 45

Fig. 44

Fig. 46

*Fig. 43*

EP 2 323 603 B1

850

*Fig. 47*

850

868

870

872

*Fig. 48*

*Fig. 49*

*Fig. 50*

*Fig. 52*

*Fig. 51*

CHOICE ALGORITHM

*Fig. 53a*

*Fig. 53b*

CHOICE ALGORITHM

20-30
(mm Hg)

| CLASSIC |
| FREEDOM |
| LITE |

30-40
(mm Hg)

| CLASSIC |
| FREEDOM |
| LITE |

20    25    30    35    40    45
ANKLE CIRCUMFERENCE (cm)

*Fig. 53c*

COLOR INDICIA

| | |
|---|---|
| GREEN | 15 - 20 mm Hg |
| YELLOW | 20 - 30 mm Hg |
| ORANGE | 30 - 40 mm Hg |
| RED | 40 - 50 mm Hg |

*Fig. 53d*

*Fig. 54*

A-D | below knee

R:  L:  | Up 5 cm circumference
R:  L:  | Up 5 cm circumference
R:  L:  | Up 5 cm circumference
R:  L:  | Up 5 cm circumference
R:  L:  | Up 5 cm circumference
R:  L:  | B circumference
R:  L:  | A$^1$ circumference

R:  L:
Measure
posteriorly

least ankle

mid-foot

A = Floor

Straight distance X

R:  L:  | x straight distance

| Circumference | 20-25 | 25-30 | 30-35 | 35-40 | 40-45 | 45-50 | 50-55 | 55-60 | 60-65 | 65-70 | 70-75 |
|---|---|---|---|---|---|---|---|---|---|---|---|
| Classic | $X_1$ | $X_2$ | $X_3$ | $X_4$ | $X_5$ | $X_6$ | $X_7$ | $X_8$ | $X_9$ | $X_{10}$ | $X_{11}$ |
| LITE | $Y_1$ | $Y_2$ | $Y_3$ | $Y_4$ | $Y_5$ | $Y_6$ | $Y_7$ | $Y_8$ | $Y_9$ | $Y_{10}$ | $Y_{11}$ |
| Freedom | $Z_1$ | $Z_2$ | $Z_3$ | $Z_4$ | $Z_5$ | $Z_6$ | $Z_7$ | $Z_8$ | $Z_9$ | $Z_{10}$ | $Z_{11}$ |

Legpiece:
Measure circumference at point B, and then measure 5 additional circumferences going up the leg by 5 cm increments. Measure A-D length following the posterior contour of the leg. Find the TTF footpiece size under which the measurements fall. Details: Small TTF legpiece fits between 35.5 cm and 38.5 cm length and leg circumference between 18 - 56 cm. If length is between 38.5 and 44 cm, apply the enclosed extra band to the top of the wrap. If length > 44 cm or circumference > 56 cm, a large TTF legpiece will be required. Do not apply LITE TTF legpiece on circumferences less than 18 cm, as it may cut off circulation to very small diameter limbs.

Starting from the distal most band (bottom) of the legpiece, cut each unstretched band the same length as the corresponding circumference measurement. You may round up to the nearest 5 cm.

Footpiece
Measure midfoot circumference. Measure distance X. Find the TTF footpiece size under which the measurements fall. Trim the correctly matched TTF footpeice length and midfoot circumference to be the same as the related measurements. Otherwise needs custom.

70cm 60cm 50cm 40cm 30cm 20cm    20cm 30cm 40cm 50cm 60cm 70cm

70cm 60cm 50cm 40cm 30cm 20cm    20cm 30cm 40cm 50cm 60cm 70cm
70cm 60cm 50cm 40cm 30cm 20cm    20cm 30cm 40cm 50cm 60cm 70cm
60cm 50cm 40cm 30cm 20cm    20cm 30cm 40cm 50cm 60cm
50cm 40cm 30cm 20cm    20cm 30cm 40cm 50cm
50cm 40cm 30cm 20cm    20cm 30cm 40cm 50cm
40cm 30cm 20cm    20cm 30cm 40cm

*Fig. 55*

SOFT TISSUE

BONY OR TENDONOUS AREA

$$\frac{C'' - C'}{C'}$$

$$\frac{B'' - B'}{B'}$$

$$\frac{A'' - A'}{A'}$$

*Fig. 56*

*Fig. 57a*

*Fig. 57b*

*Fig. 57c*

*Fig. 58*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6755052 B
- US 10975590 B
- US 73399107
- US 97559004